# EUROPEAN PATENT SPECIFICATION

(11) **EP 4 025 598 B1**
(45) Date of publication and mention of the grant of the patent: **08.07.2026**
(21) Application number: 20772170.5
(22) Date of filing: 02.09.2020
(51) Int. Cl.: C07K 14/725, A61P 35/00, A61P 31/00, C12N 15/62

(54) **PROTEINS COMPRISING T-CELL RECEPTOR CONSTANT DOMAINS**
PROTEINE MIT KONSTANTEN DOMÄNEN DES T-ZELL-REZEPTORS
PROTÉINES COMPRENANT DES DOMAINES CONSTANTS DE RÉCEPTEUR DE LYMPHOCYTES T

(30) Priority: 06.09.2019 US 201962896958 P
(43) Date of publication of application: 13.07.2022
(73) Proprietor: Eli Lilly and Company, Indianapolis, IN 46285 (US); The University of North Carolina at Chapel Hill, Chapel Hill, NC 27514 (US)
(72) Inventor: DEMAREST, Stephen J., Indianapolis, Indiana 46206-6288 (US); FRONING, Karen Jean, Indianapolis, Indiana 46206-6288 (US); KUHLMAN, Brian Arthur, Chapel Hill, North Carolina 27516 (US); MAGUIRE, Jack Barton, Chapel Hill, North Carolina 27516 (US)
(74) Representative: Eli Lilly and Company Limited
(86) International application number: PCT/US2020/048979
(87) International publication number: WO 2021/046072

(56) References cited:
- EP-A1- 3 015 477
- WO-A1-2018/234319
- WO-A1-2019/057122
- WO-A1-2019/067805
- WO-A2-03/020763
- DATABASE Geneseq [online] 13 June 2019 (2019-06-13), "Human TCRbeta protein mutant, SEQ:15060.", XP055746510, retrieved from EBI accession no. GSP:BGF64309 Database accession no. BGF64309
- KRISTIN STØEN GUNNARSEN ET AL: "Soluble T-cell receptor design influences functional yield in an E. coli chaperone-assisted expression system", PLOS ONE, vol. 13, no. 4, 12 April 2018 (2018-04-12), pages e0195868, XP055747047, DOI: 10.1371/journal.pone.0195868
- KAREN FRONING ET AL: "Computational stabilization of T cell receptors allows pairing with antibodies to form bispecifics", NATURE COMMUNICATIONS, vol. 11, no. 1, 11 May 2020 (2020-05-11), XP055747026, DOI: 10.1038/s41467-020-16231-7

## Description

The present disclosure relates to proteins comprising T-cell receptor (TCR) constant domains with one or more stabilization mutations, nucleic acids encoding such proteins, and methods of making and using such proteins.

T cell receptors (TCRs) are the adaptive immune system's tool for recognizing and eliminating "non-self" intracellular antigens. These non-self antigens can emerge from viral infection or from genetic alteration. Genetic alterations are key to aberrant cellular function and can lead to diseases such as cancer. TCRs exist in α/β and γ/δ forms, which are structurally similar but express on different T cells. The α/β TCRs are expressed on both CD8+ effector and CD4+ helper T cells and recognize proteosomally degraded foreign antigens displayed on infected/cancerous cell surfaces when complexed with HLA/MHC (Davis, et al., Nature, 1988. 334(6181): 395-402; Heemels, et al., Annu Rev Biochem, 1995. 64: 463-91). When expressed on CD8+ T effector cells, α/β TCRs interact specifically with Type I MHC/peptide complexes and this interaction induces T cell activation and elimination of cells displaying recognizable non-self antigens.

Structurally, the extracellular portion of native α/β TCR consists of two polypeptides, α chain and β chain, each of which has a membrane-proximal constant domain (Cα or Cβ domain), and a membrane-distal variable domain (Vα or Vβ domain). Each of the constant and variable domains includes an intra-chain disulfide bond. The variable domains contain the highly variable loops analogous to the complementarity determining regions (CDRs) of antibodies. CDR3 of the TCR interacts with the peptide presented by MHC (major histocompatibility complex), and CDR1 and CDR2 interact with the peptide and the MHC. The diversity of TCR sequences is generated via somatic rearrangement of linked variable (V), diversity (D), joining (J), and constant (C) genes; and such rearrangement generates an incredible diversity that enables the TCR to recognize diverse peptide antigens displayed by MHCs.

Many approaches have been developed to harness the exquisite non-self-recognizing properties of the α/β TCRs for therapeutic use. Recombinant α/β TCRs have been transduced/transfected into bulk naive T cells as a means of redirecting these T cells to target tumor associated antigens (Riley, et al., Nat Rev Drug Discov, 2019. 18(3):175-196; Parkhurst, et al., Clin Cancer Res, 2017. 23(10): 2491-2505). Alternately, the use of the soluble extracellular region of the TCR fused to an scFv that binds an activating T cell receptor, commonly CD3ε, has been used to redirect endogenous T cells to target tumor cells (Liddy, et al., Nat Med, 2012. 18(6): 980-7). Unlike typical BiTE-like bispecific antibodies that rely on the direct recognition of overexpressed antigens on the cell surface (Baeuerle, et al., Cancer Res, 2009. 69(12): 4941-4), TCR or TCR-mimic bispecifics can recognize a much larger subset of intracellular and abnormal tumor or viral antigens and thus have broader potential applicability (Liddy, et al., Nat Med, 2012. 18(6): 980-7).

However, TCR assembly and expression is challenging (Wilson, et al., Curr Opin Struct Biol, 1997. 7(6): 839-48). For both research and therapeutic purposes, the common method of soluble α/β TCR production has been through the expression as inclusion bodies in *Escherichia coli (E. coli)* followed by resolubilization, refolding/assembly/oxidation, and finally purification at relatively low yields (van Boxel, et al., J Immunol Methods, 2009. 350(1-2): 14-21). To simplify the assembly piece, some researchers have tried using only the variable domain regions of TCRs in a single chain format or scTv, like an antibody single chain Fv (scFv), for targeting specific HLA/peptide complexes (Stone, et al., Methods Enzymol, 2012. 503: 189-222). While scFvs have shown a propensity for instability, aggregation, and low solubility, scTCR variable domains have generally shown worse expression and stability issues. Great efforts have been made to stabilize scVα/Vβ proteins for therapeutic and diagnostic use (Stone, et al., Methods Enzymol, 2012. 503: 189-222). Unfortunately, the high diversity of Vα/Vβ germlines (much higher than V_{H}/V_{L} germlines of antibodies), renders each set of stabilizing mutations within a scVα/Vβ to be unique to the individual Vα/Vβ subunit and unlikely to find general use across multiple TCRs.

Given that most extracellular proteins are intrinsically glycosylated with complex disulfide pairings, mammalian expression is used to generate soluble TCRs. Industrial antibody production has predominately moved to mammalian expression systems (Shukla, et al., Bioeng Transl Med, 2017. 2(1): 58-69). However, α/β TCRs express poorly with less reliable assembly than antibodies when expressed in the commonly utilized Chinese hamster ovary (CHO) cells system. Many novel bispecific antibody formats, including those with relevance to soluble α/β TCR bispecifics, may require TCRs to express at antibody-like levels for proper molecular assembly, which is an obstacle for their production. Bispecific ImmTac moieties that recombinantly fuse soluble TCRs to antibody scFvs are typically expressed as insoluble inclusion bodies in bacteria, solubilized, refolded and assembled at low yield (Liddy, et al., Nat Med, 2012. 18(6): 980-7). Additionally, these moieties have intrinsically rapid serum clearance as they lack a recycling mechanism.

WO 2019/067805 A1 (UNIV SOUTHERN CALIFORNIA); discloses mutant TCR beta proteins wherein Seq ID NO 15060 has a Lysine at reside 134 (Kabat numbering). WO 2019/057122 A1 (WUXI BIOLOGICS SHANGHAI CO LTD) discloses the generation of mutations in the constant domains of the TCR alpha and TCR beta chains. These changes result in the generation of additional sulphur bridges which stabilize the complex.

There exists a need for TCR stabilization engineering that is suitable for general use across different TCRs and have good expression and/or assembly level. The present invention provides a protein comprising a first polypeptide comprising a T cell receptor (TCR) alpha constant domain (Cα) comprising at least one of the following residues: phenylalanine at position 139, isoleucine at position 150, threonine at position 190 (residues numbered according to Kabat numbering); and/or a second polypeptide comprising a TCR beta constant domain (Cβ) comprising at least one of the following residues: arginine at position 139, proline at position 155, aspartic acid or glutamic acid at position 170 (residues numbered according to Kabat numbering), wherein the protein has a higher unfolding temperature (Tm) compared to a protein comprising the same amino acid sequence except that: the Cα domain comprises serine at position 139, threonine at position 150, and alanine at position 190 (residues numbered according to Kabat numbering); and/or the Cβ domain comprises glutamic acid at position 134, histidine at position 139, aspartic acid at position 155, and serine at position 170 (residues numbered according to Kabat numbering).

Disclosed herein are proteins comprising one or more stabilization mutations in the TCR constant domains (Cα/Cβ domains) that increase the unfolding temperature of the TCR constant domains and improve the expression and/or assembly of TCRs. Disclosed herein are proteins comprising: a first polypeptide comprises a T cell receptor (TCR) alpha constant domain (Cα) comprising at least one of the following residues: phenylalanine at position 139, isoleucine at position 150, threonine at position 190 (residues numbered according to Kabat numbering); or a second polypeptide comprises a TCR beta constant domain (Cβ) comprising at least one of the following residues: lysine at position 134, arginine at position 139, proline at position 155, aspartic acid or glutamic acid at position 170 (residues numbered according to Kabat numbering).

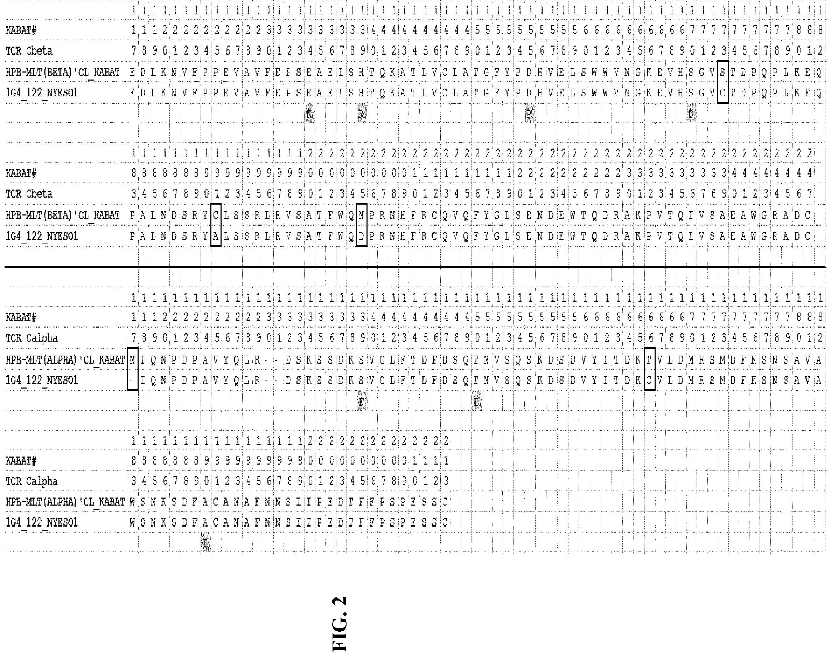

The numbering of the amino acid residues in the TCR Cα and Cβ domains used herein follows the Kabat numbering system (Kabat, et al. Sequences of Immunological Interest Vol. 1 Fifth Edition 1991 US Department of Health and Human Services, Public Health Service, NIH), as illustrated in Fig. 2. The TCR Cα residues recited above based on Kabat numbering correspond to the following residues in SEQ ID NO: 5 or 6 (Cα): position 139 of Kabat numbering corresponds to position 22 of SEQ ID NO: 5 or 6; position 150 of Kabat numbering corresponds to position 33 of SEQ ID NO: 5 or 6; position 190 of Kabat numbering corresponds to position 73 of SEQ ID NO: 5 or 6. The TCR Cβ residues recited above based on Kabat numbering correspond to the following residues in SEQ ID NO: 7 or 8 (Cβ): position 134 of Kabat numbering corresponds to position 18 of SEQ ID NO: 7 or 8; position 139 of Kabat numbering corresponds to position 23 of SEQ ID NO: 7 or 8; position 155 of Kabat numbering corresponds to position 39 of SEQ ID NO: 7 or 8; position 170 of Kabat numbering corresponds to position 54 of SEQ ID NO: 7 or 8.

In one aspect, disclosed herein are proteins comprising: a first polypeptide comprising a TCR Cα comprising at least one of the following residues: phenylalanine at position 139, isoleucine at position 150, threonine at position 190 (residues numbered according to Kabat numbering); and/or a second polypeptide comprising a TCR Cβ comprising at least one of the following residues: lysine at position 134, arginine at position 139, proline at position 155, aspartic acid or glutamic acid at position 170 (residues numbered according to Kabat numbering).

In another aspect, disclosed herein are proteins comprising a first polypeptide and a second polypeptide, wherein the first polypeptide comprises a TCR Cα comprising at least one (e.g., one, two, or three) of the following residues: phenylalanine at position 139, isoleucine at position 150, threonine at position 190 (residues numbered according to Kabat numbering); and/or the second polypeptide comprises a TCR Cβ comprising at least one (e.g., one, two, three, or four) of the following residues: lysine at position 134, arginine at position 139, proline at position 155, aspartic acid or glutamic acid at position 170 (residues numbered according to Kabat numbering). In some embodiments, the protein comprises any one of the seven residues listed above. In some embodiments, the protein comprises any two of the seven residues listed above. In some embodiments, the protein comprises any three of the seven residues listed above. In some embodiments, the protein comprises any four of the seven residues listed above. In some embodiments, the protein comprises any five of the seven residues listed above. In some embodiments, the protein comprises any six of the seven residues listed above. In some embodiments, the protein comprises all seven residues listed above.

In some embodiments, the proteins described herein are soluble proteins. In some embodiments, the proteins described herein have one or more superior properties, e.g., a higher unfolding temperature (Tm), increased stability, increased expression level when expressed under the same condition, or reduced glycosylation level, when compared to a protein comprising the same amino acid sequence except that: the Cα domain comprising serine at position 139, threonine at position 150, and alanine at position 190 (residues numbered according to Kabat numbering); and the Cβ domain comprising glutamic acid at position 134, histidine at position 139, aspartic acid at position 155, and serine at position 170 (residues numbered according to Kabat numbering).

In some embodiments, the first polypeptide comprises a Cα domain comprising phenylalanine at position 139 (Kabat numbering). In some embodiments, the first polypeptide comprises a Cα domain comprising threonine at position 190 (Kabat numbering). In some embodiments, the first polypeptide comprises a Cα domain comprising isoleucine at position 150 (Kabat numbering). In some embodiments, the second polypeptide comprises a Cβ domain comprising proline at position 155 (Kabat numbering). In some embodiments, the second polypeptide comprises a Cβ domain comprising aspartic acid or glutamic acid at position 170 (Kabat numbering). In some embodiments, the second polypeptide comprises a Cβ domain comprising lysine at position 134 (Kabat numbering). In some embodiments, the second polypeptide comprises a Cβ domain comprising arginine at position 139 (Kabat numbering).

In some embodiments, disclosed herein are proteins comprising a first polypeptide and a second polypeptide, wherein the first polypeptide comprises a TCR Cα domain comprising the following residues: phenylalanine at position 139, isoleucine at position 150, threonine at position 190 (residues numbered according to Kabat numbering); and the second polypeptide comprises a TCR Cβ domain comprising the following residues: lysine at position 134, arginine at position 139, proline at position 155, aspartic acid at position 170 (residues numbered according to Kabat numbering).

In some embodiments, disclosed herein are proteins comprising a first polypeptide and a second polypeptide, wherein the first polypeptide comprises a TCR Cα domain comprising the following residues: phenylalanine at position 139 and threonine at position 190 (residues numbered according to Kabat numbering); and the second polypeptide comprises a TCR Cβ domain comprising the following residues: lysine at position 134, arginine at position 139, proline at position 155, aspartic acid at position 170 (residues numbered according to Kabat numbering).

In some embodiments, the TCR Cα domain comprises SEQ ID NO: 5; and the TCR Cβ domain comprises SEQ ID NO: 7. Accordingly, provided herein are proteins comprising a first polypeptide comprising a Cα domain comprising SEQ ID NO: 5, and a second polypeptide comprising a Cβ domain comprising SEQ ID NO: 7. In some embodiments, the Cα domain consists of SEQ ID NO: 5; and the Cβ domain consists of SEQ ID NO: 7. Accordingly, provided herein are proteins comprising a first polypeptide comprising a Cα domain consisting of SEQ ID NO: 5, and a second polypeptide comprising a Cβ domain consisting of SEQ ID NO: 7.

In some embodiments, the first polypeptide is linked to the second polypeptide by one or more inter-chain disulfide bonds. Disulfide bonds can be formed by pairs of engineered cysteine residues in the TCR α and β chains, and such disulfide bonds link the TCR α and β chains together (see WO03/020763, WO2004/033685, WO2004/074322, Li, et al., Nat. Biotechnol. 2005, 23(3): 349-354; Boulter, et al., Protein Eng. 2003, 16(9): 707-11). For example, disulfide bonds can be formed between the following pairs of engineered cysteines at the specified residues in the TCR α and β chains: Thr48Cys (α chain) and Ser57Cys (β chain), Thr45Cys (α chain) and Ser 77Cys (β chain), Tyr10Cys (α chain) and Ser 17Cys (β chain), Thr45Cys (α chain) and Asp59Cys (β chain), and Ser15Cys (α chain) and Glu15Cys (β chain) (see WO03/020763).

In some embodiments, the Cα domain further comprises a cysteine residue at position 166 (residue numbered according to Kabat numbering), and the Cβ domain further comprises a cysteine residue at position 173 (residue numbered according to Kabat numbering), wherein the first polypeptide and the second polypeptide are linked by an inter-chain disulfide bond between the cysteine residue at position 166 of Cα and the cysteine residue at position 173 of Cβ.

In some embodiments, the TCR Cα domain comprises SEQ ID NO: 6; and the TCR Cβ domain comprises SEQ ID NO: 8. Accordingly, provided herein are proteins comprising a first polypeptide comprising a Cα domain comprising SEQ ID NO: 6, and a second polypeptide comprising a Cβ domain comprising SEQ ID NO: 8. In some embodiments, the Cα domain consists of SEQ ID NO: 6; and the Cβ domain consists of SEQ ID NO: 8. Accordingly, provided herein are proteins comprising a first polypeptide comprising a Cα domain consisting of SEQ ID NO: 6, and a second polypeptide comprising a Cβ domain consisting of SEQ ID NO: 8.

In some embodiments, the first polypeptide further comprises a TCR α chain variable domain (Vα); and the second polypeptide further comprises a TCR β chain variable domain (Vβ), wherein the Vα and Vβ form an antigen binding domain that binds an antigen, e.g., a tumor antigen or a viral antigen. In some embodiments, the Vα is fused to the N-terminus of Cα, forming a Vα - Cα domain; and the Vβ is fused to the N-terminus of Cβ, forming a Vβ - Cβ domain.

The TCR variable regions (Vα/Vβ) can bind any tumor or viral antigen, including but not limited to, a viral antigen, a neoantigen (the antigens expressed only in cancer cells but not in normal cells), a tumor-associated antigen (the processed fragments of proteins that are expressed at low levels in normal cells, but are overexpressed in cancer cells), or a cancer/testis (CT) antigen (derived from proteins usually only expressed by reproductive tissues, e.g. testes, fetal ovaries, and placenta, and have limited/no expression in all other adult tissues) (see Pritchard, et al., BioDrugs, 2018, 32:99-109).

In some embodiments, the TCR variable region (Vα/Vβ) binds an antigen selected from any one of the following: ERBB2, CD19, NY-ESO-1, MAGE (e.g., MAGE-A1, A2, A3, A4, A6, A10, A12), gp100, MART-1/Melan-A, gp75/TRP-1, TRP-2, Tyrosinase, BAGE, CAMEL, SSX-2, β-Catenin, Caspase-8, CDK4, MUM-2 (TRAPPC1), MUM-3, MART-2, OS-9, p14ARF (CDKN2A), GAS7, GAPDH, SIRT2, GPNMB, SNRP116, RBAF600, SNRPD1, PRDX5, CLPP, PPP1R3B, EF2 (see Pritchard, et al., BioDrugs, 2018, 32:99-109; and Wang, et al., Cell Research, 2017, 27:11-37).

In some embodiments, the proteins described herein further comprise a second antigen binding domain. Such second antigen binding domain can bind an antigen on the T cell surface, e.g., CD3, CD4, CD8. In some embodiments, the second antigen binding domain binds CD3.

In some embodiments, the second antigen binding domain is an antibody or antibody fragment, e.g., an scFv, Fab, Fab', (Fab')₂, single domain antibody, or camelid VHH domain. In some embodiments, the second antigen binding domain is a Fab. In some embodiments, the Fab comprises a Fab heavy chain comprising a heavy chain variable domain (VH) and a human IgG CH1 domain, and a Fab light chain comprising a light chain variable domain (VL) and a human light chain constant domain (CL), wherein the VH and VL domains form the second antigen binding domain that binds an antigen on the T cell surface, e.g., CD3, CD4, CD8.

In some embodiments, the proteins described herein comprise three polypeptides: a first polypeptide comprising Vα - Cα - linker- VH - CH1; a second polypeptide comprising Vβ - Cβ; and a third polypeptide comprising VL - CL; wherein the second and third polypeptides are linked to the first polypeptide by inter-chain disulfide bonds. In some embodiments, the proteins described herein have a TCR-CD3 Fab format.

In some embodiments, the proteins described herein comprise four polypeptides: a first polypeptide comprising Vα - Cα - hinge - first Fc region; a second polypeptide comprising Vβ - Cβ; a third polypeptide comprising VL - CL; and a fourth polypeptide comprising VH - CH1 - hinge - second Fc region; wherein the second and fourth polypeptides are linked to the first polypeptide by inter-chain disulfide bonds; and the third polypeptide is linked to the fourth polypeptide by inter-chain disulfide bonds. In some embodiments, the proteins described herein have an IgG like format or TCR-CD3 Fab-Fc IgG format.

In some embodiments, the proteins described herein comprise four polypeptides: a first polypeptide comprising Vα - Cα - linker - VH- CH1 - hinge - first Fc region; a second polypeptide comprising Vβ - Cβ; a third polypeptide comprising VL-CL; a fourth polypeptide comprising a second Fc region; and wherein the second, third, and fourth polypeptides are linked to the first polypeptide by inter-chain disulfide bonds. In some embodiments, the proteins described herein have a TCR-CD3 Fab-Fc tandem format.

In some embodiments, the proteins described herein comprise a VHH domain that binds human serum albumin (HSA). In some embodiments, the proteins described herein comprise three polypeptides: a first polypeptide comprising Vα - Cα - linker- VH - CH1-VHH; a second polypeptide comprising Vβ - Cβ; and a third polypeptide comprising VL - CL; wherein the second and third polypeptides are linked to the first polypeptide by inter-chain disulfide bonds. In some embodiments, the proteins described herein have a TCR-CD3 Fab-VHH format.

In some embodiments, the proteins described herein comprise two TCR Vα - Cα domains and two TCR Vβ - Cβ domains. In some embodiments, the proteins described herein have a 2xTCR-CD3 Fab-Fc format.

In some embodiments, the proteins described herein comprise a linker comprising an amino acid sequence selected from any one of SEQ ID NOs: 41-46.

In some embodiments, the proteins described herein comprise an Fc region, e.g., a human IgG Fc region, e.g., a human IgG1, IgG2, IgG3, or IgG4 Fc region. In some embodiments, the Fc region is a modified human IgG Fc region with reduced effector function compared to the corresponding wild type human IgG Fc region.

In some embodiments, the Fc region is a modified human IgG1 Fc region. IgG1 is well known to bind to the proteins of the Fc-gamma receptor family (FcyR) as well as C1q. Interaction with these receptors can induce antibody-dependent cell cytotoxicity (ADCC) and complement-dependent cytotoxicity (CDC). Therefore, certain amino acid substitutions are introduced into human IgG1 Fc region to ablate immune effector function. In some embodiments, the Fc region is a modified human IgG1 Fc region comprising one or more of the following mutations: N297A, N297Q, D265A, L234A, L235A, C226S, C229S, P238S, E233P, L234V, P238A, A327Q, A327G, P329A, K322A, L234F, L235E, P331S, T394D, A330L, M252Y, S254T, T256E (residues numbered according to the EU Index Numbering). In some embodiments, the Fc region is a modified human IgG1 Fc region comprising the following mutations: L234A, L235A and N297Q (residues numbered according to the EU Index Numbering). In some embodiments, the Fc region is a modified human IgG1 Fc region comprising SEQ ID NO: 48. In some embodiments, the proteins described herein further comprise a human IgG1 hinge region (e.g., SEQ ID NO: 47), at the N-terminus of the modified human IgG1 Fc region.

In some embodiments, the Fc region is a modified human IgG4 Fc region comprising one or more of the following mutations: E233P, F234V, F234A, L235A, G237A, E318A, S228P, L236E, S241P, L248E, T394D, M252Y, S254T, T256E, N297A, N297Q (residues numbered according to the EU Index Numbering). In some embodiments, the Fc region is a modified human IgG4 Fc region comprising the following mutations: F234A and L235A (residues numbered according to the EU Index Numbering). In some embodiments, the Fc region is a modified human IgG4 Fc region comprising SEQ ID NO: 50. In some embodiments, the proteins described herein further comprise a modified human IgG4 hinge region comprising the S228P mutation (according to the EU Index Numbering, e.g., SEQ ID NO: 49), at the N-terminus of the modified human IgG4 Fc region. Such modified human IgG4 hinge region reduces the IgG4 Fab-arm exchange *in vivo* (see Labrijn, et al., Nat Biotechnol 2009, 27(8):767).

In some embodiments, the proteins described herein comprise a hinge region comprising SEQ ID NO: 47 or 49. In some embodiments, the proteins described herein comprise an Fc region comprising SEQ ID NO: 48 or 50. In some embodiments, the proteins described herein comprise a hinge region comprising SEQ ID NO: 47 and a first and second Fc region comprising SEQ ID NO: 48. In some embodiments, the proteins described herein comprise a hinge region comprises SEQ ID NO: 49 and a first and second Fc region comprising SEQ ID NO: 50.

In some embodiments, the first and second Fc regions comprise a set of heterodimerization mutations, e.g., a set of CH2 and/or CH3 heterodimerization mutations. In some embodiments, the first and second Fc regions comprise a set of CH3 heterodimerization mutations, e.g., knobs-in-holes (Ridgway, et al., Protein Eng. 1996, 9:617-621), electrostatic mutations, and other CH3 dimerization mutations described in Verdino, et al., Current Opinion in Chemical Engineering 2018, 19:107-123; WO2016118742; US Patent Nos. 9605084, 9701759, 10106624; US Patent Application Publication No. 20180362668.

In some embodiments, one of the first or second Fc region comprises a CH3 domain comprising an alanine at residue 407; and the other of the first or second Fc region comprises a CH3 domain comprising a valine or methionine at residue 366 and a valine at residue 409 (residues numbered according to the EU Index Numbering). In some embodiments, one of the first or second Fc region comprises a CH3 domain comprising an alanine at residue 407, a methionine at residue 399, and an aspartic acid at residue 360; and the other of said first or second Fc region comprises a CH3 domain comprising a valine at residue 366, a valine at residue 409, and an arginine at residues 345 and 347 (residues numbered according to the EU Index Numbering).

In some embodiments, the proteins described herein are linked to a detectable label. In some embodiments, such detectable label can be a fluorescent label, a radioactive label, a chemiluminescent label, a bioluminescent label, a paramagnetic label, an MRI contrast agent, an organic dye, or a quantum dot.

In some embodiments, the proteins described herein are linked to a therapeutic agent, e.g., a cytotoxic agent, an anti-inflammatory agent, or an immunostimulatory agent.

In some embodiments, the proteins described herein are soluble proteins. In some embodiments, the proteins described herein are transmembrane proteins.

In some embodiments, the first polypeptide of the protein further comprises the transmembrane and intracellular domains of the TCR α chain; and the second polypeptide further comprises the transmembrane and intracellular domains of the TCR β chain.

Also provided herein are T cells comprising a TCR that comprises a Cα domain comprising at least one (e.g., one, two, or three) of the following residues: phenylalanine at position 139, isoleucine at position 150, threonine at position 190 (residues numbered according to Kabat numbering); and/or a Cβ domain comprising at least one (e.g., one, two, three, or four) of the following residues: lysine at position 134, arginine at position 139, proline at position 155, aspartic acid or glutamic acid at position 170 (residues numbered according to Kabat numbering). Such TCR can also include an antigen binding domain, e.g., an antibody fragment or TCR Vα / Vβ domain.

In another aspect, provided herein are nucleic acids encoding a polypeptide of the proteins described herein. Such nucleic acid can encode a polypeptide comprising a TCR Cα domain comprising at least one (e.g., one, two, or three) of the following residues: phenylalanine at position 139, isoleucine at position 150, threonine at position 190 (residues numbered according to Kabat numbering). Such a nucleic acid can encode a polypeptide comprising a TCR Cβ domain comprising at least one (e.g., one, two, three, or four) of the following residues: lysine at position 134, arginine at position 139, proline at position 155, aspartic acid or glutamic acid at position 170 (residues numbered according to Kabat numbering). In some embodiments, provided herein are nucleic acids encoding a polypeptide comprising SEQ ID NO: 5 or 7. In some embodiments, provided herein are nucleic acid encoding a polypeptide comprising SEQ ID NO: 6 or 8.

In another aspect, provided herein are vectors comprising nucleic acids encoding a polypeptide of the proteins described herein. Such vectors can further include an expression control sequence operably linked to the nucleic acid encoding a polypeptide of the proteins described herein. Expression vectors capable of direct expression of genes to which they are operably linked are well known in the art. Expression vectors can encode a signal peptide that facilitates secretion of the polypeptides from a host cell. The signal peptide can be an immunoglobulin signal peptide or a heterologous signal peptide. The expression vectors are typically replicable in the host organisms either as episomes or as an integral part of the host chromosomal DNA. Expression vectors can contain selection markers, e.g., tetracycline, neomycin, and dihydrofolate reductase, to permit detection of those cells transformed with the desired DNA sequences. The vectors containing the polynucleotide sequences of interest can be transferred into the host cell by well-known methods (e.g., stable or transient transfection, transformation, transduction or infection), which vary depending on the type of cellular host. In some embodiments, provided herein are vectors comprising a nucleic acid encoding a polypeptide comprising SEQ ID NO: 5 and a nucleic acid encoding a polypeptide comprising SEQ ID NO: 6. In some embodiments, provided herein are vectors comprising a nucleic acid encoding a polypeptide comprising SEQ ID NO: 7 and a nucleic acid encoding a polypeptide comprising SEQ ID NO: 8.

Also provided herein are host cells, e.g., mammalian cells, comprising a nucleic acid or vector described herein; such host cells can express the proteins described herein. Mammalian host cells known to be capable of expressing functional proteins include CHO cells, HEK293 cells, COS cells, and NSO cells. The present disclosure further provides a process for producing a protein described herein by cultivating the host cell described above under conditions such that the protein is expressed, and recovering the expressed protein.

In another aspect, provided herein are pharmaceutical compositions comprising a protein, nucleic acid, vector, or cell described herein. Such pharmaceutical compositions can also comprise one or more pharmaceutically acceptable carriers, diluents, or excipients.

In another aspect, provided herein are methods of treating cancer or infection in a subject in need thereof by administering to the subject a therapeutically effective amount of a protein, nucleic acid, vector, cell, or pharmaceutical composition described herein.

Also provided are proteins, nucleic acids, vectors, cells, and pharmaceutical compositions described herein for use in a therapy. Furthermore, the present disclosure also provides proteins, nucleic acids, vectors, cells, or pharmaceutical compositions described herein for use in the treatment of cancer or infection. Additionally, the present disclosure provides the use of a protein, nucleic acid, vector, cell, or pharmaceutical composition described herein in the manufacture of a medicament for the treatment of cancer or infection.

As used herein, the term "a," "an," "the" and similar terms used in the context of the present invention (especially in the context of the claims) are to be construed to cover both the singular and plural unless otherwise indicated herein or clearly contradicted by the context.

The term "antibody," as used herein, refers to monoclonal immunoglobulin molecules comprising four polypeptide chains, two heavy (H) chains and two light (L) chains interconnected by disulfide bonds. Each heavy chain comprises a heavy chain variable region (VH) and a heavy chain constant region (CH). The heavy chain constant region comprises three domains, CH1, CH2, and CH3. Each light chain is comprised of a light chain variable region (VL) and a light chain constant region (CL). The VH and VL regions can be further subdivided into regions of hypervariability, termed complementarity determining regions (CDRs), interspersed with regions that are more conserved, termed framework regions (FR). Each VH and VL is composed of three CDRs and four FRs, arranged from amino-terminus to carboxyl-terminus in the following order: FR1, CDR1, FR2, CDR2, FR3, CDR3, FR4. The CDR regions in VH are termed HCDRl, HCDR2, and HCDR3. The CDR regions in VL are termed LCDRl, LCDR2, and LCDR3. The CDRs contain most of the residues which form specific interactions with the antigen. Assigning the residues to the various CDRs may be done by algorithms, such as Kabat, Chothia, or North. The Kabat CDR definition (Kabat et al., "Sequences of Proteins of Immunological Interest," National Institutes of Health, Bethesda, Md. (1991)) is based upon antibody sequence variability. The Chothia CDR definition (Chothia et al., "Canonical structures for the hypervariable regions of immunoglobulins", Journal of Molecular Biology, 196, 901-917 (1987); Al- Lazikani et al., "Standard conformations for the canonical structures of immunoglobulins", Journal of Molecular Biology, 273, 927-948 (1997)) is based on three-dimensional structures of antibodies and topologies of the CDR loops. The North CDR definition (North et al., "A New Clustering of Antibody CDR Loop Conformations", Journal of Molecular Biology, 406, 228-256 (2011)) is based on affinity propagation clustering with a large number of crystal structures.

The term "antigen-binding domain" or "antibody fragment" refers to a portion of an antibody that retains the ability to specifically interact with (e.g., by binding, steric hinderance, stabilizing/destabilizing, spatial distribution) an epitope of an antigen. Examples of antibody fragments include, but are not limited to, Fab, Fab', F(ab')2, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody.

The term "bind" as used herein means the ability of a protein or molecule to form a type of chemical bond or attractive interaction with another protein or molecule, which results in the close proximity of the two proteins or molecules as determined by common methods known in the art.

The term "Fc region" as used herein refers to a polypeptide comprising the CH2 and CH3 domains of a constant region of an immunoglobulin, e.g., IgG1, IgG2, IgG3, or IgG4. Optionally, the Fc region may include a portion of the hinge region or the entire hinge region of an immunoglobulin, e.g., IgG1, IgG2, IgG3, or IgG4. In some embodiments, the Fc region is a human IgG Fc region, e.g., a human IgG1 Fc region, IgG2 Fc region, IgG3 Fc region or IgG4 Fc region. In some embodiments, the Fc region is a modified IgG Fc region with reduced effector function compared to the corresponding wild type IgG Fc region. The numbering of the residues in the Fc region is based on the EU index as in Kabat. Kabat et al, Sequences of Proteins of Immunological Interest, 5th edition, Bethesda, MD: U.S. Dept. of Health and Human Services, Public Health Service, National Institutes of Health (1991). The boundaries of the Fc region of an immunoglobulin heavy chain might vary, and the human IgG heavy chain Fc region is usually defined as the stretch from the amino acid residue at position 231 (according to the EU index) to the carboxyl-terminus of the immunoglobulin.

The term "polypeptide", as used herein, refers to a polymer of amino acid residues. The term applies to polymers comprising naturally occurring amino acids and polymers comprising one or more non-naturally occurring amino acids.

The term "therapeutically effective amount," as used herein, refers to an amount of a protein or nucleic acid or vector or cell or composition of the invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of a protein or nucleic acid or vector or cell or composition of the invention that, when administered to a subject, is effective to at least partially alleviate, inhibit, prevent and/or ameliorate a condition, or a disorder or a disease.

As used herein, "treatment" or "treating" refers to all processes wherein there may be a slowing, controlling, delaying or stopping of the progression of the disorders disclosed herein, or ameliorating disorder symptoms, but does not necessarily indicate a total elimination of all disorder symptoms. Treatment includes administration of a protein or nucleic acid or vector or cell or composition of the present invention for treatment of a disease or condition in a patient, particularly in a human. References to methods of treatment in this description are to be interpreted as references to the compounds, pharmaceutical compositions and medicaments of the present invention for use in a method for treatment of the human (or animal) body by therapy (or for in vivo diagnosis).

### Brief Description of the Drawings:

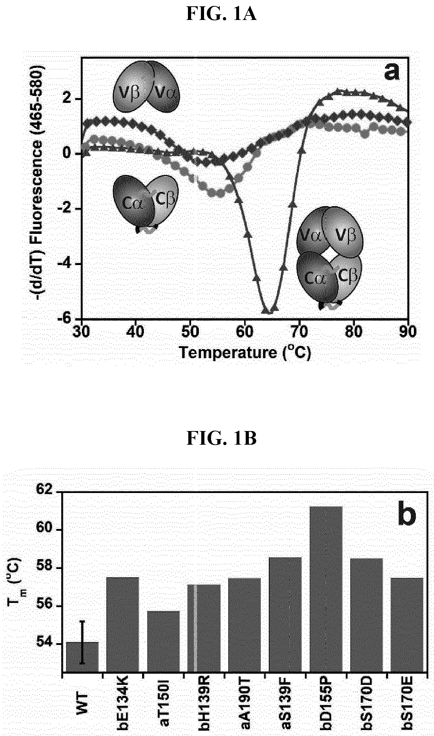

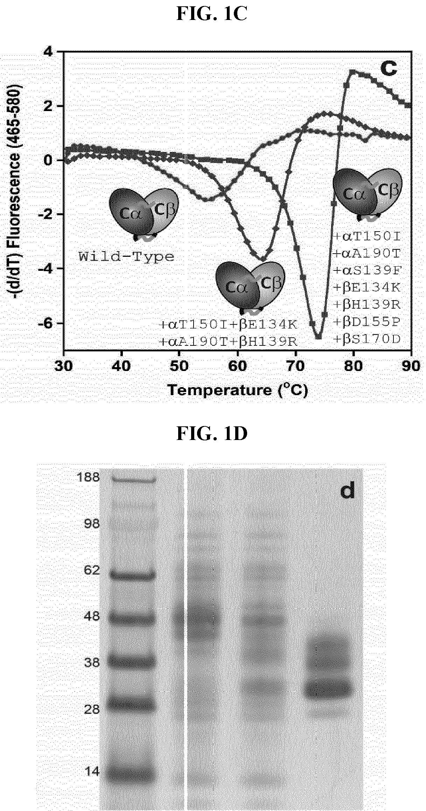

**Figs. 1A-1D** show the characterization of TCR fragments and stabilizing mutations. **Fig. 1A** shows DSF denaturation curves with a recombinant TCR (1G4_122, A), Vα/Vβ fragment from the same TCR (◆) with an α42/β110 engineered disulfide (homologous to the VH44/VL100 disulfide in dsFvs), and a Cα/Cβ fragment **(•)** with both the standard stalk disulfide and a stabilizing α166/β173 disulfide. **Fig. 1B** is **a** bar graph showing the midpoints of thermal unfolding (Tₘs) of the 'wild-type' (WT) Cα/Cβ subunit and the Cα/Cβ subunit with indicated individual stabilizing mutations. **Fig. 1C** shows DSF curves of wild-type Cα/Cβ (•) and Cα/Cβ with 4 (◆) or 7 **(■)** stabilizing mutations. **Fig. 1D** shows the SDS-PAGE characterization of wild-type Cα/Cβ (left lane) and Cα/Cβ with 4 (middle lane) or 7 (left lane) stabilizing mutations.
**Fig. 2** shows the Kabat numbering system of the TCR Cβ and Cα domains based on two different TCRs. The upper Cβ and Cα sequences were derived from the first human T-lymphocyte receptor Human HPB-MLT sequences in Kabat, et al., (Sequences of Immunological Interest Vol. 1 Fifth Edition 1991 US Department of Health and Human Services, Public Health Service, NIH, pages 1004 and 1005) (human HPB-MLT Cβ, SEQ ID NO: 51; human HPB-MLT Cα, SEQ ID NO: 52); and the bottom 1G4_122 NYESOI1 Cβ and Cα sequences (1G4_122_NYESO1 Cβ, SEQ ID NO: 4; 1G4_122_NYESO1 Cα, SEQ ID NO: 3) were derived from the 2F53 pdb crystal structure (Dunn, et al., Protein Sci 2006. 15: 710-21). Boxed squares indicate differences between the constant domains of 2F53 and the HPB-MLT native sequences. Cβ_S173C and Cα_T166S in the 2F53 sequence are the cysteines that form the stabilizing disulfide bond (Boulter, et al., Protein Eng. 2003. 16: 707-11). The shaded residues underneath the alignment are the stabilizing mutations described here that dramatically stabilize the Cα/Cβ subunit and the different full-length TCRs that harbor the stabilized Cα/Cβ subunit.

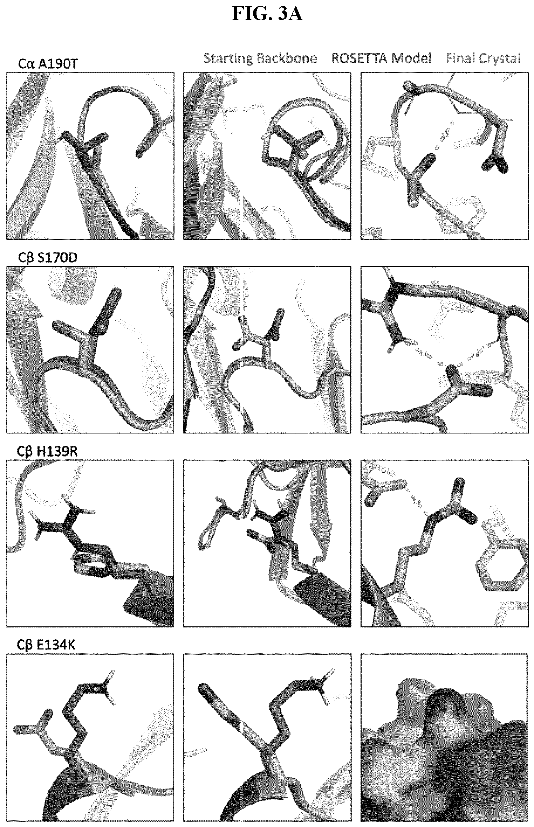

**Fig. 3A** shows the structural characterization of mutations involving polar amino acids. Each row features a single mutation. The first (left) column features a zoomed in schematic ribbon diagram of the Cα/Cβ backbone structure along with a stick depiction of the original residue that is mutated superimposed with the structural diagram of the model harboring the stabilizing mutation. The second (middle) column shows a superposition of the stabilizing mutation within the Cα/Cβ model diagram superimposed upon the crystal structure diagram of the same mutation. The third column (right) column highlights a feature of that mutation that makes it stabilizing.

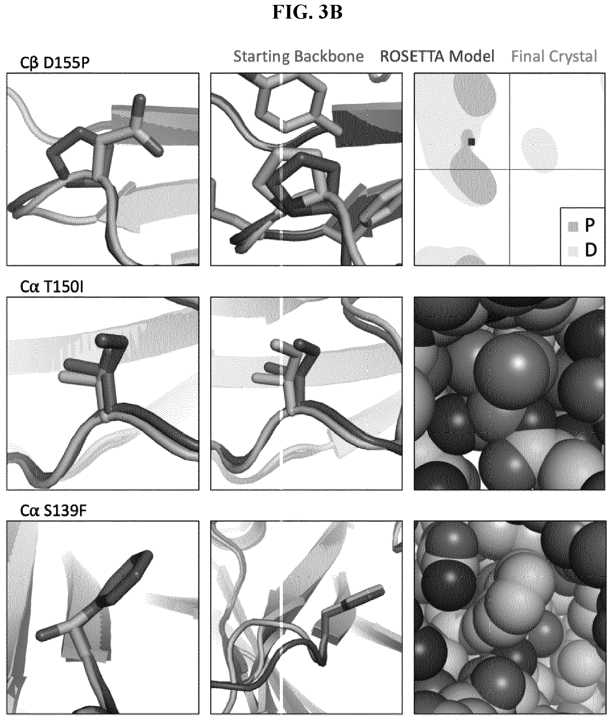

**Fig. 3B** shows the structural characterization of mutations involving hydrophobic amino acids. Each row features a single mutation. The first (left) column features a zoomed in schematic ribbon diagram of the Cα/Cβ backbone structure along with a stick depiction of the original residue that is mutated superimposed with the structural diagram of the model harboring the stabilizing mutation. The second (middle) column shows a superposition of the stabilizing mutation within the Cα/Cβ model diagram superimposed upon the crystal structure diagram of the same mutation. The third column (right) column highlights a feature of that mutation that makes it stabilizing. The third column of the first row shows the Ramachandran plot for proline overlaid on the Ramachandran plot for aspartic acid with Cβ 155 represented by the dark box. The plot shows that Cβ D155 naturally adopts a backbone phi/psi dihedral angle preferred by proline. The third column of the following two rows exhibit how well the mutations pack with surrounding residues, with Cα T150I slightly darker to make it more visible.

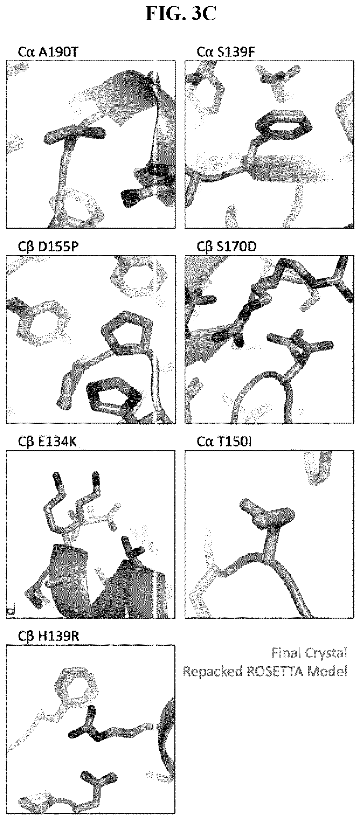

**Fig. 3C** shows comparison of the crystal structure of the 7-mutant TCR and the output of Rosetta when it is used to predict the conformations of the amino acid side chains given the backbone coordinates of the 7-mutant Cα/Cβ structure.

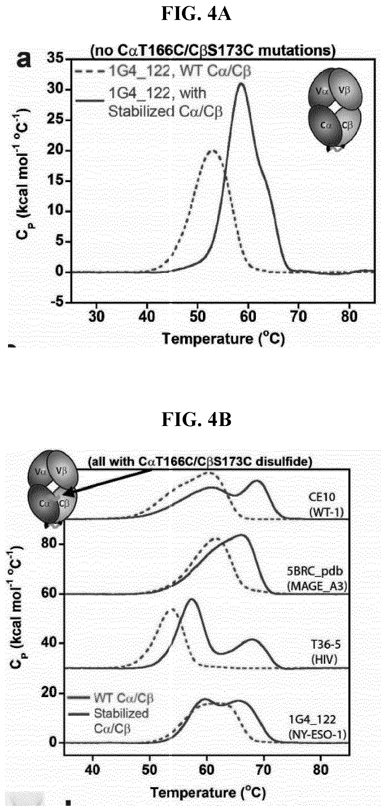

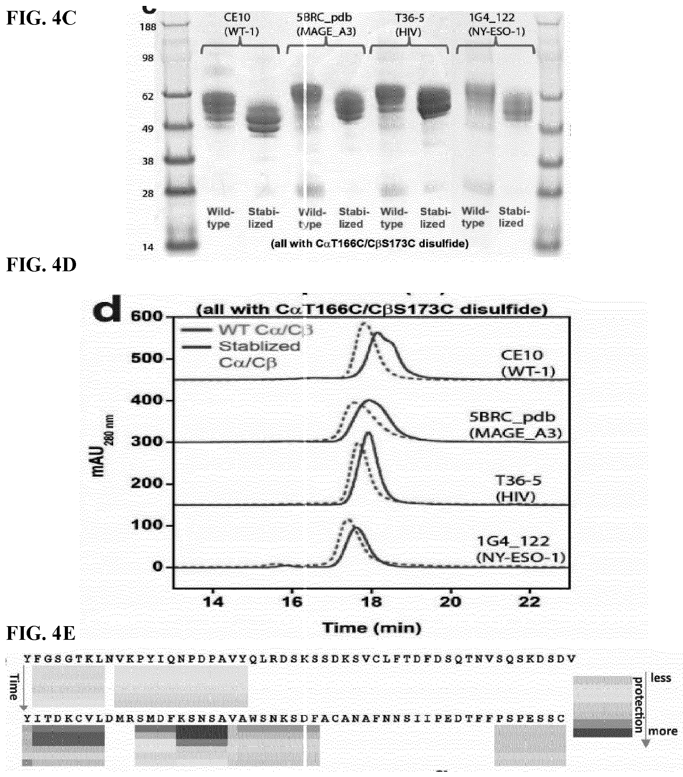

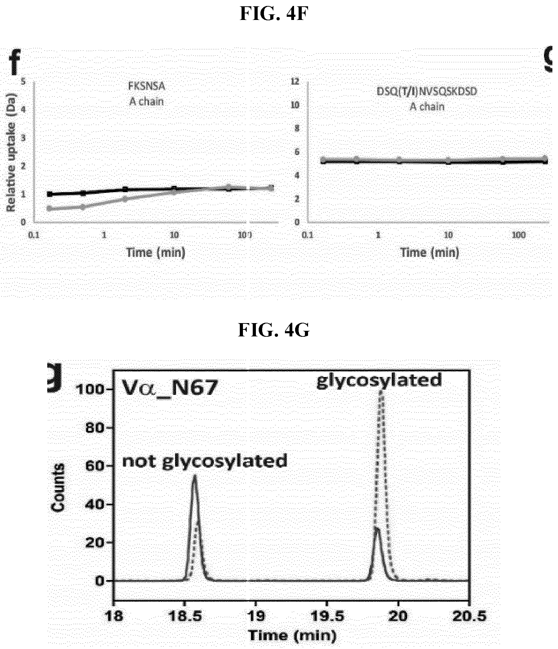

**Figs. 4A-4G** show characterization of four diverse, recombinant α/β TCRs with and without a stabilized Cα/Cβ subunit. **Fig. 4A** shows DSC curves of the 1G4_122 TCR with zero (WT, dotted line) or seven (solid line) stabilizing Cα/Cβ mutations in the absence of the α166/β173 stabilizing disulfide. **Fig. 4B** shows DSC curves of four, diverse TCRs with zero (WT, dotted line) or seven (solid line) stabilizing mutations all in the presence of the α166/β173 disulfide. **Fig. 4C** shows the non-reduced SDS-PAGE of four, diverse TCRs with zero (WT, dotted line) or seven (solid line) stabilizing mutations all in the presence of the α166/β173 disulfide. **Fig. 4D** shows the analytical SEC of four, diverse TCRs with zero (WT, dotted line) or seven (solid line) stabilizing mutations all in the presence of the α166/β173 disulfide. **Fig. 4E** shows the only significant areas of backbone protection from deuterium exchange were in Cα. The heat map under each peptide region indicates the level of protection observed at 10s, 30s, 2min, 10min, 1hr, and 4hr. Lack of a heat map indicates no significant differences in deuterium exchange observed in the region. **Fig. 4F** shows representative deuterium uptake plots of peptides from the wild-type CE10 TCR (black squares) and stabilized CE10 TCR (grey circles). **Fig. 4G** shows extracted ion chromatograms of the peptide containing the Vα_N67 N-linked glycosylation site. The peptide at 18.6 min was not glycosylsated while the peak 19.9 min shows up after enzymatic deglycosylation/conversion to Asp. The peptide from the wild-type TCR is a dotted line and the peptide from the stabilized TCR is a solid line.

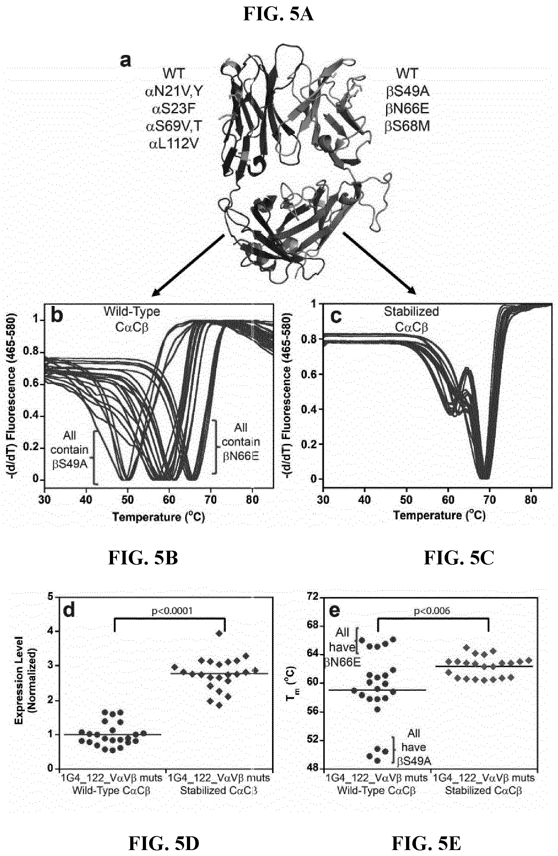

**Figs. 5A-5E** show the characterization of both stabilizing and destabilizing Vα/Vβ mutants within the 1G4_122 TCR with or without the stabilized Cα/Cβ subunit. **Fig. 5A** shows cartoon depiction of the 1G4_122 (pdb 2F53) with various Vα/Vβ mutations shown in lavender. DSF curves of the 1G4_122 TCR in the presence of various Vα/Vβ mutant combinations and in the absence **(****Fig. 5B****)** and presence **(****Fig. 5C****)** of a stabilized Cα/Cβ subunit. Normalized expression levels **(****Fig. 5D****)** and lowest Tm **(****Fig. 5E****)** of the 1G4_122 TCR with different variable domain mutations in the absence (•) and presence (◆) of a stabilized Cα/Cβ subunit.

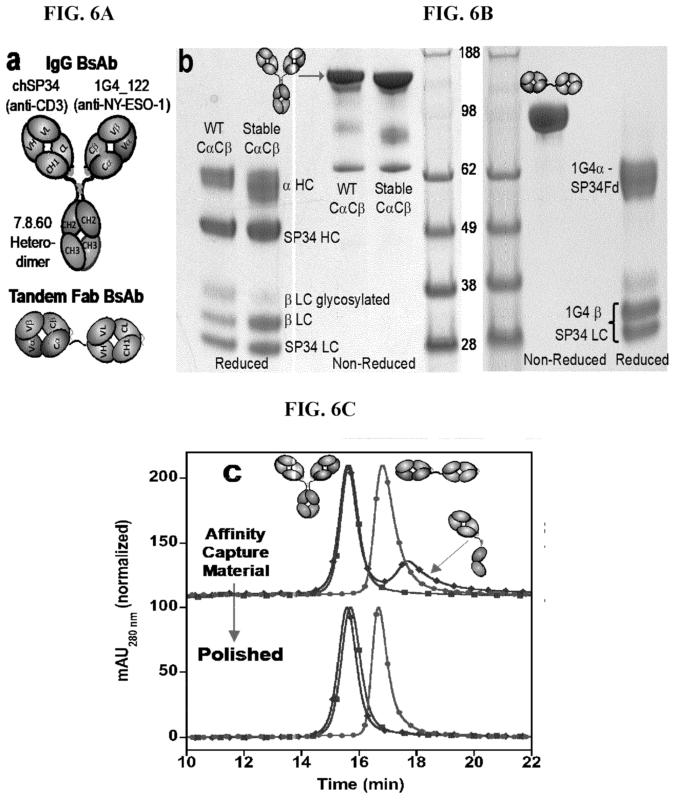

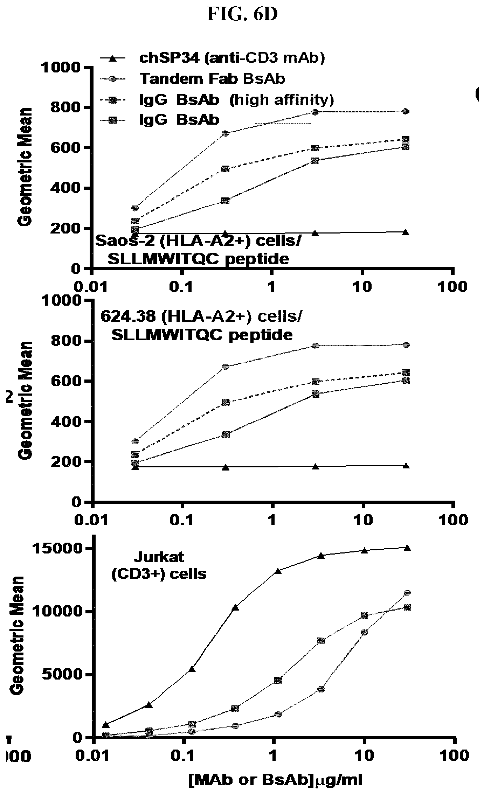

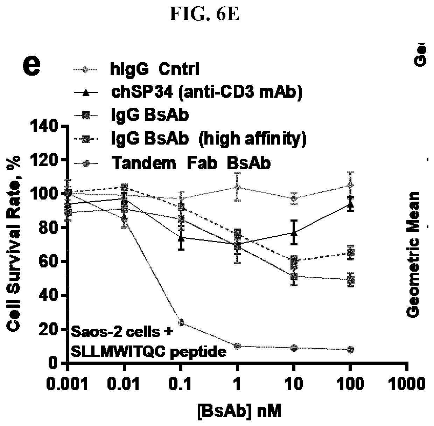

**Figs. 6A-6E** show the characterization of IgG-like and Tandem-Fab like TCR/CD3 proteins. **Fig. 6A** is a schematic diagram of the TCR/CD3 IgG-like and Tandem-Fab like bispecific molecules. **Fig. 6B** shows non-reduced and reduced SDS-PAGE of TCR/CD3 IgG-like and Tandem-Fab like bispecific proteins. **Fig. 6C** shows analytical SEC characterization of the TCR/CD3 IgG-like BsAbs using the wild-type NY-ESO-1 TCR and a stabilized NY-ESO-1 TCR as well as a tandem Fab BsAb using only the stabilized NY-ESO-1. **Fig. 6D** shows flow cytometry cell binding titrations of the BsAb molecules to Saos-2 (top) and 624.38 (middle) HLA-A2+ tumor cells pulsed with the NY-ESO-1 peptide and to Jurkat (bottom) CD3+ cells. **Fig. 6E** shows T cell redirected killing of Saos-2 tumor cells pulsed with NY-ESO-1 peptide.

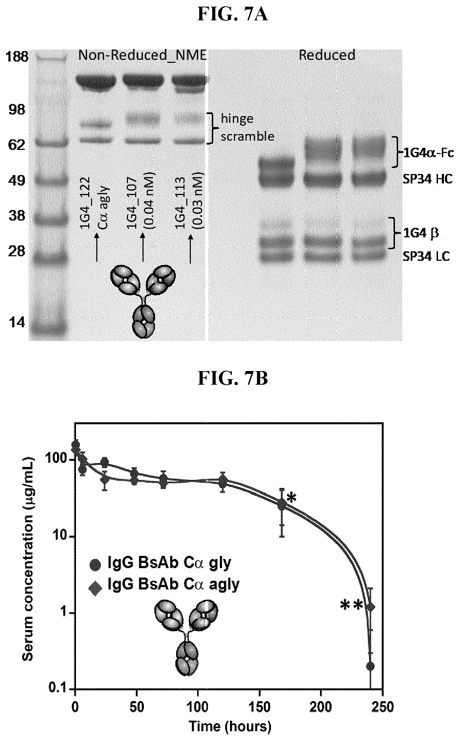

**Figs. 7A-7B** shows additional TCR/CD3 bifunctionals and mouse pharmacokinetics of the IgG-like TCR/CD3 BsAbs. **Fig. 7A** shows the reduced and non-reduced SDS-PAGE of the IgG-llike TCR/CD3 BsAbs with different affinity or with Cα deglycosylated by mutation of the canonical N-linked glycosylation sites. **Fig. 7B** shows the serum concentrations of the 1G4_122/SP34 IgG-like BsAb with or without the N-linked glycosylation in Cα following a single 5 mg/kg intravenous injection in Balb-c mice.

### EXAMPLES

Recombinant TCRs can be used to redirect naive T cells to eliminate virally infected or cancerous cells; however, they are plagued by low stability and uneven expression. Molecular modeling is used to identify mutations in the TCR constant domains (Cα/Cβ subunits) to improve Cα/Cβ stability, increase Cα/Cβ expression, and increase the unfolding temperature of Cα/Cβ. When 7 of these mutations are collectively added to the Cα/Cβ subunit, an increase in the midpoint of thermal unfolding by 20 °C is observed. Adding the stabilized Cα/Cβ subunit improves the expression and assembly of four separate α/β TCRs by 3- to 10-fold. Additionally, the mutations rescued the expression of TCRs with destabilizing mutations in the variable domains. Interestingly, the improved stability and folding of the TCRs led to reduced glycosylation. The Cα/Cβ variant enabled antibody-like expression, allowing development of a new class of bispecific molecules that combine an anti-CD3 antibody with the stabilized TCR. These TCR/CD3 bispecific proteins can redirect T cells to kill tumor cells expressing the HLA/peptide antigens.

General stabilization of the Cα/Cβ subunit may improve the overall stability and folding of α/β TCRs. Recent studies have shown that strong thermodynamic cooperativity exists between the subunits of α/β TCRs. Cα requires pairing with Cβ in the ER for folding similar to what has been observed for antibody C_{H}1/Cκ subunits (Feige, et al., Mol Cell, 2009. 34(5): 569-79; Toughiri, et al., MAbs, 2016. 8(7): 1276-1285). Additionally, many Vα/Vβ subunits are intrinsically unfolded in isolation and require the Cα/Cβ subunit for proper folding (Feige, M.J., et al., J Biol Chem, 2015. 290(44): p. 26821-31). In support of the hypothesis, adding a disulfide between the Cα/Cβ domains has been shown to have a positive impact on many α/β TCRs (Boulter, J.M., et al., Protein Eng, 2003. 16(9): p. 707-11). Therefore, a more robust Cα/Cβ subunit is engineered for general TCR stabilization with the goal of generating TCRs at antibody-like levels that assemble properly. A stabilized TCR should be a more amenable building block for recombinant fusion to antibodies in different geometries including those that include an antibody-Fc moiety to enhance their pharmacokinetic properties.

Protein simulations were performed with the molecular modeling software Rosetta to identify mutations that stabilize the Cα and Cβ domains (Leaver-Fay, et al., Methods Enzymol, 2011. 487: 545-74). An alternative strategy for finding mutations that will stabilize a protein is to assemble a multiple sequence alignment (MSA) for the protein family and search for highly conserved amino acids that are not conserved in the protein of interest (Magliery, et al., Curr Opin Struct Biol, 2015. 33: 161-8). Here, mutations were tested based on Rosetta calculations as well as use conservation analysis to filter the results from the simulations.

### Results

### Stabilizing the Cα/Cβ TCR subunit

First, the thermodynamic properties of the α/β TCR are examined by generating a soluble form of the α/β TCR, 1G4_122, and its Vα/Vβ and Cα/Cβ subunits; 1G4_122 binds to the NY-ESO-1 antigen (Li, Y., et al., Nat Biotechnol, 2005. 23(3): 349-54). Using a mammalian expression system, both the Vα/Vβ and Cα/Cβ subunits were generated in the presence or absence of flexible (Gly4Ser)4 linkers (SEQ ID NO: 43) that link Vα to Vβ or Cα to Cβ. The subunit expression and assembly were tested with or without stabilizing interdomain disulfides. Most of the Vα/Vβ and Cα/Cβ constructs either failed to express or failed to assemble, including the single chain variants. The best Vα/Vβ subunit expression was obtained by adding a Vα44/Vβ110 disulfide homologous to the V_{H}44/V_{L100} disulfide used to stabilize antibody variable domain fragments or Fvs (Brinkmann, et al., Proc Natl Acad Sci USA, 1993. 90(16): 7538-42), while the best Cα/Cβ expression was obtained using the known stabilizing disulfide (Cα166/Cβ173) on top of the native Cα/Cβ disulfide at the C-terminus of the domains (Cα213/Cβ247) (Boulter, et al., Protein Eng, 2003. 16(9): 707-11). Differential scanning calorimetry (DSC) experiments showed the (Cα166/Cβ173) disulfide increased the midpoint of thermal unfolding (Tₘ) of the full length TCR (both subunits) by 8 °C. When comparing the Vα/Vβ and Cα/Cβ subunit unfolding to the unfolding of the intact extracellular TCR domain, the individual subunits were clearly both destabilized in the absence of one another **(****Fig. 1A****),** agreeing with the thermodynamic cooperativity observed previously (Feige, et al., J Biol Chem, 2015. 290(44): 26821-31).

Molecular modeling simulations were also used to identify mutations that stabilize the constant domains. For each simulation, the TCR was fixed in space and only residues in close proximity to the mutation were allowed to make small movements ("relax") to accommodate the mutation. These local relaxations were repeated using the native sequence without any mutations. The change in energy was determined by comparing the Rosetta score of the mutation with the Rosetta score of the native sequence. The mutations were split into two lists: a list of mutations that are observed in a multiple sequence alignment (MSA) of TCRs and a list of mutations that are absent from the MSA. The mutations with the best predicted energies from both lists were picked for experimental screening.

Using the isolated Cα/Cβ fragment containing the Cα166/Cβ173 disulfide bond **(****Fig. 1A****),** the library of single, computationally selected mutants was generated and expressed in two separate blocks. Increased expression level was used blindly to select mutants for scale-up, purification, and characterization by differential scanning fluorimetry (DSF, **Table 1).** Increased expression correlated well with protein stability. Roughly half of the single mutants chosen for further evaluation based on expression demonstrated a significant increase in stability over the wild-type (native) Cα/Cβ protein **(Table 1).** The Tₘs for seven of the identified stabilizing mutants are shown in **Fig. 1B** and range from +1 to +7 °C over wild-type Cα/Cβ. Five of these mutations were substitutions that are observed in a multiple sequence alignment of TCRs, but the two mutations that produced the biggest gains in Tₘ, D155P (+7.1 °C) and S139F (+4.4 °C), are not observed in the multiple sequence alignment.

Next, the stabilizing mutations were combined into a variant harboring four of the mutations and a variant with all seven mutations. Modeling results suggested each of the mutations was likely compatible with each another (i.e., unlikely to interfere with each other). Including all seven stabilizing mutations led to a 7-fold increase in expression and a 20 °C increase in the Tₘ over the Cα/Cβ subunit harboring only the Cα166/Cβ173 disulfide (denoted 'wild-type') **(Table 2,** **Fig. 1C****).** Assembly of the wild-type and mutant Cα/Cβ subunits was probed on a non-reducing SDS-PAGE gel. The Cα/Cβ variant with seven mutations assembled more efficiently as evidenced by the lack of disulfide laddering and was smaller/more compact, likely due to less spurious glycosylation (described in detail below) via maintainance of a more discretely folded and compact structure **(****Fig. 1D****).** Even for the stabilized variant, multiple bands are clear on the SDS-PAGE gel **(****Fig. 1D****)** that likely reflect partial glycan occupancy of the three N-linked glycosylation sites in Cα and single N-linked glycosylation site in Cβ.

A primary sequence depiction of the exact locations of each mutation along with their proper numbering according to Kabat notation (Kabat, et al. Sequences of Immunological Interest Vol. 1 Fifth Edition 1991 US Department of Health and Human Services, Public Health Service, NIH) is shown in **Fig. 2****.**

**Table 1. Expression of Cα/Cβ subunits with various mutations derived by modeling.**

| Mutation (NY-ESO-1 peptide numbering) | Mutation (Kabat numbering) | Titer (mg/L) | Titer Ratio (Variant/ matched WT) | DSF Tₘ (°C) | Rosetta Predicted dE (REU) | Present in MSA | Region |
|---|---|---|---|---|---|---|---|
| Cα/Cβ wild type | | 12.8 | 1.00 | 54.9 | | | |
| βP173A | βP178A | 10.4 | 0.81 | | -1.61 | + | Core |
| βE129K | βE134K | 14.1 | 1.10 | 57.5 | -2.34 | + | Surface |
| αR126K | αR129K | 17.5 | 1.37 | 53.1 | -0.81 | + | Interface |
| αV122L | αV125L | 12.4 | 0.97 | | -1.17 | + | Surface |
| βA123D | βA128D | 10.4 | 0.81 | | -1.50 | + | Interface |
| βK115S | βK120S | 13.8 | 1.08 | 53.7 | -1.56 | + | Surface |
| αD183S | αD188S | 16.1 | 1.26 | 53.3 | 0.66 | + | Surface |
| αV176L | αV181L | 11.3 | 0.88 | | -2.10 | + | Interface |
| αT145I | αT150I | 13.6 | 1.06 | 55.7 | -1.66 | + | Surface |
| βH134R | βH139R | 15 | 1.17 | 57.1 | -3.11 | + | Interface |
| βQ199H | βQ204H | 11.5 | 0.90 | | -3.11 | + | Interface |
| βN116K | βN121K | 5.31 | 0.41 | | -1.16 | + | Surface |
| βE121T | βE126T | 10.8 | 0.84 | | -1.30 | + | Interface |
| αD154E | αD159E | 12.2 | 0.95 | | -0.25 | + | Surface |
| αA185T | αA190T | 14.6 | 1.14 | 57.5 | -1.87 | + | Surface |
| αI157A | αI162A | 8.61 | 0.67 | | 3.61 | - | Core |
| βL154A | βL159A | 7.91 | 0.62 | | 0.98 | - | Core |
| βL143A | βL148A | 1.72 | 0.13 | | 1.74 | + | Interface |
| Cα/Cβ wild type | | 21.3 | 1.00 | 53.3 | | | |
| αQ116K | αQ119K | 34.2 | 1.61 | | -2.95 | + | Surface |
| αR126F | αR129F | 8.13 | 0.38 | | -3.45 | - | Interface |
| αK129R | αK134R | 30.9 | 1.45 | | -3.93 | + | Interface |
| αS130G | αS135G | 8.64 | 0.41 | | -5.85 | - | Surface |
| αS131G | αS136G | 35.7 | 1.68 | 52.7 | -2.71 | + | Surface |
| αS134F | αS139F | 35.7 | 1.68 | 58.5 | -4.50 | - | Surface |
| αS134G | αS139G | 28.38 | 1.33 | | 0.92 | - | Surface |
| αS134Y | αS139Y | 22.95 | 1.08 | | -4.21 | - | Surface |
| αS143D | αS148D | 24.54 | 1.15 | | -4.19 | - | Surface |
| αT145Q | αT150Q | 22.74 | 1.07 | | -4.13 | - | Surface |
| αK151V | αK156V | 23.7 | 1.11 | | 0.05 | + | Surface |
| αK151A | αK156A | 59.1 | 2.77 | 54.3 | -2.76 | + | Surface |
| αK151D | αK156D | 26.55 | 1.25 | | -4.01 | - | Surface |
| αK151N | αK156N | 42 | 1.97 | 53.9 | -3.57 | - | Surface |
| αK151S | αK156S | 26.85 | 1.26 | | -3.49 | + | Surface |
| αS167Y | αS172Y | 4.62 | 0.21 | | -4.05 | - | Surface |
| αK171Y | αK176Y | 4.71 | 0.22 | | -4.18 | - | Surface |
| αN173Y | αN178Y | 21.27 | 1.00 | | -3.13 | + | Core |
| αN191Y | αN196Y | 11.88 | 0.56 | | -3.86 | - | Surface |
| βL114Y | βL119Y | 28.86 | 1.35 | | -3.83 | - | Core |
| βA123T | βA128T | 39 | 1.83 | 53.9 | -2.78 | + | Interface |
| βA123V | βA128V | 30.3 | 1.42 | | -2.99 | + | Interface |
| βT135K | βT140K | 6.48 | 0.30 | | -2.76 | + | Interface |
| βQ136G | βQ141G | 4.65 | 0.22 | | -3.26 | + | Surface |
| βK137L | βK142L | 26.19 | 1.23 | | -4.52 | - | Interface |
| βD150P | βD155P | 47.4 | 2.23 | 61.2 | -3.72 | - | Interface /Core |
| βV158I | βV163I | 27.72 | 1.30 | | -4.00 | - | Surface |
| βS165D | βS170D | 58.8 | 2.76 | 58.5 | -2.84 | + | Interface |
| βS165E | βS170E | 37.8 | 1.77 | 57.5 | -4.12 | - | Interface |
| βD170Q | βD175Q | 26.37 | 1.23 | | -3.92 | + | Interface |
| βA179W | βQ184W | 18.57 | 0.87 | | -3.49 | - | Surface |
| βA179Y | βQ184Y | 4.8 | 0.23 | | -3.44 | - | Surface |
| βS194H | βS199H | 26.01 | 1.22 | | -3.69 | - | Surface |
| βK137T | βK142T | 20.85 | 0.98 | | -4.06 | + | Interface |

| | | | | | | | |
|---|---|---|---|---|---|---|---|
| ^{a}all CαCβ fragments contain the αT166C/βS173C disulfide. | | | | | | | |

Lastly, the impact of the mutations on potential immunogenicity of the TCR proteins was assessed. The EpiVax server was used to investigate whether any increase MHC-peptide complexes were likely for each of the mutations (De Groot, et al., Clin Immunol, 2009. 131(2): 189-201). Overall, a slight increase in the EpiMatrix score was observed for both Cα and Cβ. Where increases were observed, these tended to be slight increases in the baseline scores that did not reach the level predicted to elicit significant MHC binding as described by Epibars. Where Epibars were observed in the native sequences, there were some subtle increases in the scores, but few that led to the Epibars moving into a higher category of risk. An outlier of concern includes the Cα_T150I variant, whose EpiMatrix score goes up substantially for one 9-mer and there is a strong, existing EpiMatrix cluster in a second peptide for the wild-type Cα protein that also exists for the mutant. TCRs recognizing the wild-type peptide may be eliminated during immune development and tolerance. This mutation provides the smallest contribution to the stability. The Cβ_E134K_H139R mutants are found together on multiple single peptides and there is a slight increase in the EpiMatrix scores for these two residues. The Epibars that are introduced for this double mutant are primarily in the low risk category with no increases to the high risk category. Interestingly, the Cβ_D155P and Cβ_S170D variants, which are the two most impactful mutants from a Tₘ perspective, lower the overall EpiMatrix scores versus wild-type Cα/Cβ.

**Table 2. Expression and thermal stability of wild-type (WT) TCRs and TCRs with the 7 stabilizing mutations (Des).**

| | | Titer (mg/L) | Titer Ratio (Variant/WT) | DSF Tₘ (°C) |
|---|---|---|---|---|
| | Cα/Cβ Subunit | | | |
| 31303s1 | TCR2/4 WT constants only | 7.3 | 1 | 54.4±1.0 |
| 31303s2/ 29632s1 | TCR45/46: 4 mutants | 4.2/20 | 1.66 | 64.1 |
| 31303s3 | TCR47/50 Des | 48/59 | 7.3 | 73.4 |

| | Full-Length TCRs^{a} | | | DSC Tₘ (°C) |
|---|---|---|---|---|
| 31434s1 | NY-ESO-1 WT (αT166/βS173) | 9.5 | 1 | 53.0 |
| 31434s2 | NY-ESO-1 Des (αT166/βS173) | 104 | 10.9 | 58.6, 63.8 |
| 30754s2/ 28820s1 | NY-ESO-1 WT | 24 | 1 | 60.8, 63.7 |
| 28820s2 | NY-ESO-1 with 4 mutants | 75 | 3.1 | 59.7, 65.0 |
| 30754s1 | NY-ESO-1 Des | 85 | 3.5 | 59.5, 65.2 |
| 31631s3 | MAGE_A3 WT | 29 | 1 | 61.5 |
| 31631s4 | MAGE_A3 Des | 80 | 2.76 | 61.4, 66.5 |
| 31631s5 | HIV_T36-5 WT | 30 | 1 | 53.5 |
| 31631s6 | HIV_T36-5 Des | 80 | 2.67 | 57.3, 67.6 |
| 31631s1 | WT1_CE10 WT | 25 | 1 | 55.9, 61.1 |
| 31631s2 | WT1_CE10 Des | 70 | 2.8 | 60.6, 69.0 |

| | | | | |
|---|---|---|---|---|
| ^{a}Unless stated otherwise, all TCRs contain the αT166C/βS173C disulfide. | | | | |

### Structural characterization of the seven stabilizing Cα/Cβ mutations.

Next, attempts to crystallize both the wild-type and stabilized forms of the Cα/Cβ subunit were performed. The proteins were deglycosylated enzymatically before crystallization. Consistent with the SDS-PAGE gel results with the proteins **(****Fig. 1D****),** only the stabilized version yielded protein crystals. A 1.76 Å crystal structure of the stabilized Cα/Cβ subunit was obtained. The side chains for all seven mutants are resolved in the structure and provide an indication of how the mutations are stabilizing the protein **(****Fig. 3A** **and** **Fig. 3B****)**

In the crystal structure and the Rosetta model, phenylalanine 139 (Cα S139F) fills a partially buried cleft in the structure and forms tight packing interactions with the side chain of Cα 129Q. Notably, the orientation of the interaction leaves the polar groups on the glutamine open for forming hydrogen bonds with water. Despite being solvent exposed, Cβ D155P has the largest impact on protein stability of the seven mutations. The Rosetta energy calculations favor the proline because the residue has backbone torsion angles (*ϕ* = -73.4°, *ψ* = 52.3°) compatible with the closed ring of the proline. **Fig. 3B** shows overlapping Ramachandran plots for both aspartic acid and proline and highlights that Cβ 155 is in the allowed region for both amino acids. One benefit of mutating to proline, however, is that proline is more restrictive than aspartic acid in Ramachandran space and therefore loses less entropy when the protein folds. Isoleucine 150 (Cα T150I) packs tightly with surrounding residues and removal of the threonine does not leave any buried polar groups without a hydrogen bond partner.

Cα A190T is at the beginning of a turn in a solvent-exposed loop. In addition to increasing solubility on the surface, Rosetta predicted that this threonine would form a stabilizing hydrogen-bond to Cα 129Q. Instead, the crystal structure shows the threonine adopting a different rotamer to form a hydrogen bond with the backbone on the other end of the loop's turn (backbone nitrogen of Cα 193N). Rosetta predicted that Cβ S170D would form a bidentate hydrogen bond across the interface with the sidechain of Cα 171R. The crystal structure shows Cβ S170D hydrogen bonding with the backbone nitrogen atom of Cα 171R (which was not previously participating in a hydrogen bond) and allowing the arginine's sidechain to become more solvent-exposed. Cβ H139R creates an interface-spanning hydrogen bond with Cα 124D. Rosetta modeled the arginine and aspartic acid to form a bidentate hydrogen bond, however the crystal structure shows that they form just one hydrogen bond so that the arginine can better pack with neighboring residues. The new lysine at residue 134 on the β chain (Cβ E134K) has high b-factors and is likely interacting with several negatively charged amino acids in the vicinity.

Investigation was carried out to see why Rosetta did not predict the observed sidechain conformations. One possibility is that the sidechain and backbone sampling protocol in Rosetta was unable to sample these conformations. Alternatively, Rosetta may have sampled them but predicted them to be higher in energy. To test these hypotheses, the backbone coordinates from the crystal structure of the stabilized mutant was used as the starting point for fixed-backbone sidechain prediction simulations. The backbone conformation in the new crystal structure is similar to the crystal structure of the wild-type protein, but there are small deviations that may affect sidechain positioning and ability to form hydrogen bonds. This is what has been observed, given the crystal structure backbone Rosetta correctly predicted most of the side chain conformations of the mutants **(****Fig. 3C****).** This result suggests that Rosetta did not correctly predict the sidechain conformations of these mutations in the original model because it did not sample or favor the small backbone perturbations observed in the crystal structure.

### Impact of the Cα/Cβ Mutations on Full Length TCRs.

To evaluate the impact of the Cα/Cβ mutations on the expression and stability of unrelated, full-length, soluble TCRs, the 1G4_122 anti-NY-ESO-1 TCRs were generated with and without the novel Cα/Cβ mutations. Without the disulfide, the 1G4_122 TCR expressed extremely poorly in mammalian cells, but with the Cα/Cβ mutations, the expression increased over 10-fold and the Tₘ measured by DSC was 8 °C higher **(Table 2,** **Fig. 4A****).** The stabilizing Cα/Cβ mutations were also evaluated in the presence of the CαT166C/CβS173C disulfide in four unrelated (and sequence diverse) α/β TCRs including additional TCRs targeting MAGE_A3 (pdb 5BRZ; NCBI: α=ABY74337.1/β=ACZ48691.1, HIV (pdb 3VXT; NCBI: α=ABB89050.1/β=ACY74607.1), and WT-1 antigens (Raman, et al., Sci Rep, 2016. 6: 18851; Shimizu, et al., Sci Rep, 2013. 3: 3097; Richman, S.A., et al., Mol Immunol, 2009. 46(5): 902-16). Even in the presence of the CαT166C/CβS173C disulfide, each of the TCRs experienced a roughly 3-fold increase in titer and an increase in thermal stability by DSC in the presence of the stabilized Cα/Cβ subunit **(Table 2,** **Fig.4B****).** Interestingly, all four TCRs were more compact as assessed by SDS-PAGE analysis and analytical size exclusion chromatography (SEC, **Figs. 4C-4D****).**

To investigate further the more compact nature of the TCRs in the presence of the Cα/Cβ mutations, both hydrogen deuterium exchange (HDX) analyses and glycan occupancy studies were performed using peptide mass mapping with the WT-1 TCR (CE10) with and without the stabilizing Cα/Cβ mutations. HDX patterns of the CE10 TCR with and without the Cα/Cβ mutations was nearly identical except for significant regions of the Cα chain **(****Fig. 4E****).** HDX protection in the presence of the Cα domain upon stabilization was not localized to the sites of mutation, but more globally to various regions of the Cα fold, including some protection at the C-terminus. The wild-type and stabilized CE10 TCR was also evaluated for the presence/occupancy of N-linked glycans before and after enzymatic N-linked and O-linked deglycosylation. A single N-linked glycosylation motif exists in the Vα domain, three in the Cα domain, and one in the Cβ domain. Cα/Cβ stabilization led to a significant reduction in N-linked glycosylation within the CE10 TCR including within Vα, suggesting the stabilized Cα/Cβ mutations also stabilize the Vα/Vβ domains **(Table 3,** **Fig 4F****).** O-linked glycosylation was probable at the C-terminus of Cα since the C-terminal peptide could not be resolved in the TCR lacking the stabilizing mutations. This issue was eliminated in the presence of the Cα/Cβ mutations with a well-resolved C-terminal peptide. The Cα C-terminal region is variably present in different TCR crystal structures and absent in the NY-ESO-1 structure being used. Interestingly, the Cβ H139R mutation forms charge-charge interactions as well as a potential π-stacking interaction that may stabilize/lock-down the conformation of the Cα C-terminus, which also shows enhanced protection from HDX. Thus, the universal decrease in hydrodynamic radius observed for all the stabilized TCRs by SDS-PAGE and SEC was confirmed as a reduction in glycosylation.

Next the ability of the Cα/Cβ mutations to enable TCRs to withstand variable domain mutation was assessed. Using Rosetta, a limited number of mutations in both the Vα and Vβ domains were selected. Ultimately, one (βN66E) was stabilizing, multiple mutations were neutral, and one was significantly destabilizing (βS49A) within the wild-type 1G4_122 anti-NY-ESO-1 TCR **(****Fig. 5A****).** The Vα and Vβ mutations were combined and this small library of TCR variants were expressed in the absence and presence of the Cα/Cβ stabilizing mutations. A wide range of Tₘs were observed by DSF for the 1G4_122 TCRs in the absence of the Cα/Cβ mutations, while the stability of the 1G4_122 variants was virtually constant when the Cα/Cβ mutations were present **(****Figs. 5B-5C****).** As observed with the TCRs described above, the entire library of 1G4_122 variants achieved a roughly 3-fold increase in titer with the Cα/Cβ mutations present **(****Fig. 5D****).** Interestingly, the Tₘs of the 1G4_122 variants ranged from 49 - 65 °C, whereas the Cα/Cβ stabilized library all had Tₘs tightly clustered around 62 °C **(****Fig. 5E****)** and were impervious to variable domain instability. These results suggest the Cα/Cβ mutations may rescue the expression and stability of TCRs with low intrinsic stability.

**Table 3. Glycan occupancy of the CE10 TCR in the absence and presence of the stabilizing Cα/Cβ mutations.**

| N-linked glycosylation site | %Glycan occupancy Wild-Type CE10 TCR | %Glycan occupancy Cα/Cβ Stabilized CE10 TCR |
|---|---|---|
| Vα_N67 | 78 | 30 |
| Cα_N151 | 70 | 20 |
| Cα_N185 | 65 | 5 |
| Cα_N196 | n.d.a | 80 |
| Cβ_N186 | 60 | 25 |

| | | |
|---|---|---|
| ^{a}Peptide could not be resolved. Possible O-linked glycosylation at CαT204 occludes peptide resolution. | | |

### Utility of the Cα/Cβ mutations in producing well-assembled, soluble TCR/CD3 bispecifics for redirecting T cells to target tumor cells.

A major benefit to stabilizing α/β TCRs would be the novel forms of bispecifics that could be enabled. The first generation TCR bispecifics combine the specificity of soluble α/β TCRs with an anti-CD3 scFv in the ImmTac format (Liddy, et al., Nat Med, 2012. 18(6): 980-7). This format enables exquisite redirected lysis potency; however, low yield bacterial expression and refolding as well as rapid serum clearance of this format make the *in vivo* dosing challenging. With the significant increase in expression and proper folding of α/β TCRs in mammalian cells afforded by the Cα/Cβ mutations, new bispecific formats should be accessible that include moieties with enhanced pharmacokinetics (PK) as well as avidity to the tumor cells.

IgG-like and Tandem Fab-like bispecific antibodies (BsAbs) were generated **(****Fig. 6A****).** The BsAbs utilized the 1G4_122 α/β TCR that binds the HLA-A2/NY-ESO-1 peptide complex and was engineered to bind with high affinity (1 nM) (Li, Y., et al., Nat Biotechnol, 2005. 23(3): 349-54). The IgG-like format requires the expression of two heavy chains that require a set of antibody Fc heterodimerization mutations to achieve proper heavy chain assembly. The previously published 7.8.60 heterodimer mutations were chosen (Leaver-Fay, et al., Structure, 2016. 24(4): 641-651). The TCR/CD3 IgG-like BsAb was expressed with or without the Cα/Cβ mutations. As observed with the soluble TCRs, the TCR/CD3 IgG-like BsAb containing the stabilizing Cα/Cβ mutations expressed over 2-fold higher than the construct lacking the Cα/Cβ mutations. The IgG-like BsAb protein lacking the stabilizing mutations was also found to be a mixture of TCR/CD3 BsAb and anti-CD3 half-antibody. The anti-CD3 half-antibody was observable as a low-molecular weight (LMW) peak by analytical SEC after IgG-Fc affinity purification **(****Figs. 6B-6C****).** The IgG-like BsAb and Tandem Fab-like BsAbs with the stabilizing Cα/Cβ mutations were secreted as >90% monodisperse (i.e., pure) proteins coming directly from the cell culture based on their profile after a single affinity chromatography step **(****Figs. 6B-6C****).**

Next, the pharmacokinetic (PK) properties of the TCR/CD3 IgG-like BsAbs were evaluated in mice (the Tandem Fab-like BsAbs were not tested). The residual glycosylation might result in systemic (particularly liver) uptake of the TCR/CD3 moiety by binding asialoglycan receptors (Sethuraman, et al., Curr Opin Biotechnol, 2006. 17(4): 341-6; Lepenies, et al., Adv Drug Deliv Rev, 2013. 65(9): 1271-81). Previously, a similar construct containing a non-stabilized Cα/Cβ demonstrated a substantial α-phase clearance, which might be due to asialoglycan receptor uptake (Wu, et al., MAbs, 2015. 7(2): p. 364-376). Since the Cα/Cβ mutations described here considerably reduce the level of glycosylation on soluble TCRs, the mutations may result in improved PK properties. As a control, a second IgG BsAb was generated that had the N-linked glycosylation sites within the Cα domain mutated as described previously (Wu, et al., MAbs, 2015. 7(2): p. 364-76). Interestingly, when the N-linked glycosylation sites were eliminated within non-stabilized Cα/Cβ subunits, the expression was decreased by more than an order of magnitude (Wu, et al., MAbs, 2015. 7(2): p. 364-76). Knock-out of the N-linked glycosylation sites in the stabilized Cα/Cβ construct had no impact on expression **(****Fig. 7A****).** Both the stabilized and the stabilized/aglycosyl TCR/CD3 IgG-like BsAbs were evaluated for their PK by intravenous injection into BALB/c mice **(****Fig. 7B****).** The IgG-like BsAbs demonstrated near identical PK in Balb-c mice with a β-half-life of roughly 3-8 days depending on the timepoints included in the fits. The Fc-moeity clearly leads to a long, antibody-like serum half-life and the presence of residual TCR glycosylation is not impacting the clearance to any great extent. This PK is much better than would be expected for ImmTacs. At the one week timepoint, a non-linear drop in molecule concentration for both TCR/CD3 IgG-like BsAb was observed. The drop in concentration was more dramatic at 10 days and by 14 days BsAb levels were largely below the level of detection **(****Fig. 7B****).** The sera were tested for the presence of mouse anti-drug antibodies (ADA) and a low level was observed at 7 days that increased substantially at 10 days and 14 days, perhaps explaining the accelerated clearance beginning at 7 days. The reason behind the early onset ADA is not clear.

The TCR/CD3 BsAb was next evaluated for their function. Flow cytometry was performed with Jurkat cells to assess CD3 binding and 624.38 and Saos-2 tumor cells to assess HLA-A2/peptide binding. Both the IgG-like and Tandem Fab-like BsAbs were capable of binding both the CD3 and HLA-A2/peptide cellular antigens **(****Fig. 6D****).** Both BsAbs showed a 10- to 30-fold decrease in binding potency to Jurkat cells compared to the bivalent anti-CD3 antibody due to a loss of avidity. The Tandem Fab-like BsAb appeared to bind slightly better to two separate HLA-A2+ tumor cells compared to the IgG-like BsAb. Given the weaker binding to tumor cells observed for the IgG-like BsAb, a couple higher potency TCR (Li, et al., Nat. Biotechnol, 2005, 23(3): 349-54) versions of the IgG BsAb were generated to offset the potency loss **(****Fig. 7A****).** The 1G4_107 TCR variant did demonstrate improved potency binding to tumor cells over the 1G4_122 variant by flow cytometry **(****Fig. 6D****).**

Next, the BsAbs were titrated onto peptide-labeled tumor cells in the presence of unstimulated primary T cells. Weak T cell redirected of tumor cells was observed on the Saos-2 cell line **(****Fig. 6E****)** for the IgG BsAb. Increasing the potency towards the HLA/peptide complex did not improve the weak activity **(****Fig. 6E****).** The Tandem Fab BsAb potently redirected T cells to kill Saos-2 cells.

Based on the data presented here, the novel Cα/Cβ mutations will likely improve the expression and stability of most recombinant TCRs including those with low stability variable domains. Compared to traditional bacterial methods of production in inclusion bodies followed by solubilization, refolding and purification (Wilson, Curr Opin Struct Biol, 1997. 7(6): 839-48), the mammalian expression/secretion of fully assembled/oxidized TCRs described here should dramatically increase their ease of production. The stabilizing mutations led to antibody-like expression levels with or without a stabilizing disulfide bond (Boulter, et al., Protein Eng., 2003. 16(9): p. 707-11) - an achievement not provided by the disulfide bond in isolation. This increased expression enables the robust production of various TCR/antibody bifunctional molecules. An interesting possible application would be to include stabilizing mutations within full-length TCRs transgenically induced within primary T cells. Difficulty with chain associations and expression have seen minor improvements via the introduction of the disulfide bond, the order of expression of the α/β chains, or introduction of hydrophobics in the transmembrane region (Jin, et al., JCI Insight, 2018. 3(8); Scholten, et al., Cell Oncol, 2010. 32(1-2): 43-56). It would be interesting to see if the Cα/Cβ mutations described here generally improve cell-surface TCR expression for cell-based therapy approaches.

The stabilized TCRs also displayed reduced overall glycosylation (both variable and constant domains) compared to their non-stabilized counterparts. Glycosylation can naturally stabilizes and solubilizes many different proteins including antibody Fc and, in specific cases, antibody Fab moieties (Zhou, J Pharm Sci, 2019. 108(4):1366-1377). Glycoengineering has even been used as an approach to stabilize proteins, reduce their propensity to aggregate/misfold, or improve their solubility (Zhou, J Pharm Sci, 2019. 108(4):1366-1377). The loss of glycosylation of Cα was shown to dramatically reduce the expression of IgG-like proteins containing a Cα/Cβ subunit (Wu, et al., MAbs, 2015. 7(2): 364-76). Thus, it is surprising to find that the stabilizing mutations significantly reduced the N-linked glycosylation by roughly 50% per site throughout the entire TCR. Putative O-linked glycosylation at the C-terminus of Cα was also absent in the stabilized form. It is hypothesized that stabilizing interactions reduce the conformational fluctuation of these sites within the folding/folded protein, reducing access of the enzymes to decorate the canonical N-linked motifs and O-linked site(s). This includes an N-linked site within Vα, providing further evidence that the Cα/Cβ mutations generally stabilize the entire TCR. Further, full-elimination of the N-linked glycans on Cα via mutation had no impact on protein expression within the stabilized constructs.

Clear geometrical constraints exist for the TCR/CD3 BsAb's ability to redirect T cells to lyse tumor cells. Such constraints have been demonstrated previously for bispecific antibody redirection (Bluemel, et al., Cancer Immunol Immunother, 2010. 59(8): 1197-209; Wu, et al., MAbs, 2015. 7(2): 364-76; Li, et al., Cancer Cell, 2017. 31(3): 383-395). No similar data has been shown for TCR-based therapeutics. The flexibility and spacing of the soluble TCR and anti-CD3 Fab subunits appears to be important to achieve high redirected lysis potency considering the substantial difference in overall activity and potency between the IgG-like and tandem Fab-like BsAbs containing identical soluble TCR and anti-CD3 arms. Many discussions regarding the size of the antigen and/or bispecific construct and their ability to fit within the immune synapse have been raised (Davis, et al., Nat. Immunol, 2006, 7(8): 803-9). However, differences were observed in binding to the tumor cells between the IgG-like and Tandem Fab-like BsAb formats in the *absence* of the T cells, which complicates the size/immune synapse interpretation. The individual binding characteristics to each cell type may play a bigger role in the case of these BsAbs. Overall, the significant advantages afforded by the stabilized Cα/Cβ mutations could be valuable not only for soluble TCR-based therapeutics, but also for improving recombinant α/β TCR expression in cellular systems including those that use TCRs as part of cell based therapies.

### Materials and Methods

### Molecular Biology

NY-ESO-1 1G4_122 soluble TCR, and the individual NY-ESO-1 Vα/Vβ and Cα/Cβ subunits were originally generated using gBlocks (Integrated DNA Technologies, IDT) and subcloned into mammalian expression vectors (Lonza) using recombinase cloning (InFusion, Clontech). Sequences for 1G4_122,107, and 113 were derived using the 2F53 crystal structure (Dunn, et al., Protein Sci, 2006. 15(4): 710-21) and the CDRs described elsewhere (Li, et al., Nat Biotechnol, 2005. 23(3): 349-54). A murine kappa light chain leader sequence was used to drive secretion of each protein and 8xHistags were added recombinantly to the N-termini of the α-chains. DNA sequences were derived using IDT optimized codon algorithms.

Single mutant libraries were created using a site-directed mutagenesis protocol. Briefly, the protocol employs the supercoiled double-stranded DNA vector and two synthetic oligonucleotide primers (generated at IDT) containing the desired mutation(s). The oligonucleotide primers, each complementary to opposite strands of the vector, are extended during thermal cycling by the DNA polymerase (HotStar HiFidelity Kit, Qiagen) to generate an entirely new mutated plasmid. Following temperature cycling, the product is treated with Dpn I enzyme to eliminate the methylated, non-mutated plasmid that was prepared using E. *coli* (New England BioLabs). Each newly generated mutant plasmids is then transformed into Top 10 *E. coli* competent cells (Life Technologies). Colonies were picked, cultured, miniprepped (Qiagen), and sequence verified. Mutant combinations were generally synthesized as gBlocks.

TCR/CD3 constructs were created using overlapping PCR of each fragment and recombinase cloning. The chimeric SP-34 anti-CD3 heavy and light chain sequences were published previously (Wu, et al., MAbs, 2015. 7(2): 364-76).

### Protein Expression and Purification.

α/β constructs were co-transfected for transient expression in either 24 well plates (1 mL scale) or individual flasks (100-200 mL scale) as described previously (Rajendra, et al., Biotechnol Prog, 2017. 33(2): 469-477). Supernatants were harvested for purification as previously described (Lewis, et al., Nat Biotechnol, 2014. 32(2): 191-8; Froning, et al., Protein Sci, 2017. 26(10): 2021-2038). Small scale expressions of TCR and TCR fragments were quantified using Ni²⁺-NTA tips (Pall Forte Bio) reading on the Octet RED384 System. TCR/TCR fragment His-tag proteins were purified using a Ni²⁺ immobilized resin (cOmplete^{™} His-Tag, Millipore Sigma) and an AKTA Pure system (GE Healthcare). The column was equilibrated with at least 5 column volumes of binding buffer (20mM Tris-HCl pH 8.0, 0.5M NaCl) at a flow rate of 1 mL/min for 1mL columns and 5mL/min for 5mL columns, respectively. After passing the CHO expression supernatant over the column to capture the histagged TCRs or TCR fragments, the TCR proteins were eluted with ten column volumes of elution buffer (20mM Tris-HCl pH8.0, 0.5M NaCl, 250mM Imidazole). Imidazole was later removed by passing the proteins through a preparative size exclusion column (SRT-10C SEC300) or via dialysis against a phosphate buffered saline solution (PBS) at pH 7.2-7.4 using VIVASPIN 6 concentrators with a 10,000 MW cutoff. IgG-Fc containing proteins were purified using mAbSure resin (GE Healthcare) and preparative size exclusion. Sodium dodecyl sulfate polyacrylamide gel electrophoresis (SDS-PAGE) was performed using 4-12% Bis Tris gels according to the manufacturer (Life Technologies). Reductions and alkylations were performed with 1 mM dithiothreitol (DTT) and 1 mM N-ethylmaleimide (NEM).

### Differential Scanning Fluorimetry and Calorimetry (DSF and DSC).

Protein unfolding was monitored on the Lightcycler 480II RT PCR machine (Roche) using SYPRO Orange dye (Invitrogen, S6651). Excitation and emission filters were set at 465 nm and 580 nm, respectively. The temperature was ramped from 25 °C to 95 °C at a rate of 1 °C/s. Stock solutions of protein were stored in running buffer (1 X PBS pH 7.4).

Final assay conditions were 0.2 mg/mL protein and a 500-fold Sypro dilution in running buffer. The experiment was performed by diluting the proteins to 0.4 mg/ml and diluting Sypro 250-fold, then combining the diluted protein and Sypro in equal parts in a 96-well plate (final volume: 30 µL per well). The samples were divided into 6 µL triplicates on a 384-multiwell assay plate (Roche, 04-729-749-001).

The midpoint of thermal unfolding (Tₘ) determination for each protein was performed on the LightCycler Thermal Shift Analysis Software (Roche) using the first derivative method. The analysis software smoothed the raw fluorescence data and the Tₘ was collected by determining the temperature where the upward slope of fluorescence vs. temperature was the steepest (i.e., temperature where first derivative of melt curve was maximal).

DSC data was collected and analyzed as described previously (Dong, et al., J Biol Chem, 2011. 286(6): 4703-17).

### Rosetta Energy Predictions.

The TCR crystal structure with PDB code 2F53 was used with chains A-C removed, leaving only the alpha and beta chains. The remaining structure was relaxed using the FastRelax protocol in Rosetta with coordinate constraints (energy penalties that grow as a backbone atom deviates from its starting position) on every residue's CA atom (Conway, et. al., Protein Sci, 2014. 23(1): 47-55). All possible mutations (except for mutations to cysteine) were also simulated using the FastRelax protocol. FastRelax consists of two sub-protocols: (1) fixed-backbone rotamer substitutions (i.e. side chain conformation sampling) and (2) all-atom minimization of backbone and side chain torsion angles. The first sub-protocol generates a rotamer library for each residue position and stochastically samples rotamers at user-allowed positions. The lowest-energy combination of rotamers is assigned to the protein after this sampling is repeated many times (hundreds of thousands). The second sub-protocol uses gradient-based minimization of torsion angles to relax the structure into its local minimum in the Rosetta energy landscape. The energy function REF2015 was used for this study (Park, J Chem Theory Comput, 2016, 12(12): 6201-6212; O'Meara, et al., J Chem Theory Comput, 2015. 11(2): 609-22). Certain considerations were made to prevent noise in the Rosetta energy predictions. For FastRelax's first sub-protocol, only residues within 10Å of the mutation were used to sample alternate side chain conformations. Coordinate constraints were added to all residue positions in the second sub-protocol to prevent the backbone from varying much from the starting structure. After FastRelax completed, one final run of all-atom minimization *without* coordinate constraints was performed; allowing the protein to relax into its local, unconstrainted energy minimum. Ten independent trajectories were run for every possible mutation and used the average score of the three lowest-scoring trajectories as the representative score for that mutation. The version identifier (git sha1) of our Rosetta version was 360d0b3a2bc3d9e08489bb9c292d85681bbc0cbd, released on June 4th, 2017.

### Multiple Sequence Alignment.

A list of 39 CA and 53 CB sequences were assessed by hand-curating a multiple sequence alignment (MSA) of TCR sequences from various organisms. Two lists of TCR-similar sequences were assembled by passing the sequences of the CA and CB chains of the 2F53 structure into BLAST using NCBI's database of non-redundant protein sequences (169,859,411 sequences in database). Both lists were purged by hand of any sequences that did not appear to be a TCR. The list of mutations deemed stabilizing by Rosetta were partitioned into two lists: (1) mutations present in the MSA and (2) mutations absent from the MSA. 30 mutations were selected from list 1 and 25 mutations were selected from list 2 for experimental testing. Selection decisions were made primarily by Rosetta score but some candidates were removed for the sake of increasing diversity. By splitting the mutations into two lists, mutations that were deemed lower-tier by Rosetta can be promoted if they have been shown to be compatible with any T-Cell Receptor structure.

### Modeling Cα/Cβ mutant combinations.

After the best performing point mutations were determined experimentally, Rosetta simulations were run to verify that each mutation was compatible with every other mutation. Each mutation was separately applied to the pre-relaxed 2F53 structure and ran fixed-backbone sidechain repacking. The same simulations were performed with every pair of mutations and ensured that the Rosetta energy of the pair was not dissimilar to the sum of the energies of the mutations individually.

### Protein crystallization.

Purified protein was crystallized at 8 °C using the sitting-drop vapor-diffusion method. Crystals were obtained by mixing one part protein solution at 15 mg/mL (10mM Tris-HCl pH 7.5, and 150 mM sodium chloride) with one part reservoir solution containing 23% PEG 4K, 300 mM magnesium sulfate, and 10% glycerol. Drops were immediately seeded with crushed crystals grown from 15% PEG 3350, and 100 mM magnesium formate. Single, plate-shaped crystals were observed within one week. These were harvested into a drop of reservoir solution containing 15% glycerol, and flash frozen in liquid nitrogen.

### X-ray data collection and structure determination.

Synchrotron X-ray diffraction data were collected on a single crystal at the Advanced Photon Source on beamline 31-ID-D (LRL-CAT). The resulting data were integrated using autoPROC (Vonrhein, et al., Acta Crystallogr D Biol Crystallogr, 2011. 67(Pt 4): 293-302), and merged and scaled with SCALA (Evans, Acta Crystallogr D Biol Crystallogr, 2011. 67(Pt 4): 282-92). The structure was solved by Molecular Replacement with Phaser (McCoy, et al., J Appl Crystallogr, 2007. 40(Pt 4): 658-674), using the TCR constant domains of a previously solved TCR-pMHC complex (Protein Databank accession code 2F53) as the search model (Dunn, et al., Protein Sci, 2006. 15(4): 710-21). Several rounds of model building and refinement were conducted with COOT (Emsley, et al., Acta Crystallogr D Biol Crystallogr, 2010. 66(Pt 4): 486-501) and REFMAC5 (Murshudov, et al., Acta Crystallogr D Biol Crystallogr, 2011. 67(Pt 4): p. 355-67), as implemented in the CCP4 software package (Winn, et al., Acta Crystallogr D Biol Crystallogr, 2011. 67(Pt 4): 235-42). The final model was validated with MolProbity (Williams, et al., Protein Sci, 2018. 27(1): 293-315). Additional data collection and refinement details can be found in Table 4 and Table 5.

**Table 4**

| Data Collection | | |
|---|---|---|
| Space group | | C2 |
| Cell dimensions | | |
| | a, b, c (Å) | 96.85, 59.85, 61.35 |
| | α, β, γ (°) | 90, 110.2, 90 |
| Resolution (Å) | | 29.33-1.76 (1.86-1.76)* |
| R_{merge} | | 0.057 (0.910)* |
| I/σI | | 5.6 (0.8)* |
| Completeness (%) | | 99.2 (99.1)* |
| Redundancy | | 3.8 (3.6)* |

| | | |
|---|---|---|
| *Values in parentheses are for the highest resolution shell. | | |

**Table 5**

| Refinement | | |
|---|---|---|
| No. of reflections | | 30,717 |
| R_{free} | | 0.230 |
| R_{work} | | 0.209 |
| No. of amino acids | | 217 |
| No. of atoms | | |
| | Protein | 1726 |
| | Ligand/ion | 3 |
| | Water | 175 |
| B-factors | | |
| | Protein | 30.1 |
| | Ligand/ion | 29.5 |
| | Water | 37.4 |
| RMS deviations | | |
| | Bond lengths (Å) | 0.007 |
| | Bond angles (°) | 1.242 |
| Ramachandran plot (%) | | |
| | Favored | 99.5 |
| | Allowed | 0.5 |
| | Outliers | 0.0 |

### Peptide Mapping.

The wild-type and stabilized CE10 TCRs were denatured, reduced, cysteine-alkylated, and digested for the analyses. Briefly, 100 µg of TCR variant was denatured in 6 M guanidine prior to reduction with 10 mM dithiothreitol at 37 °C for 30 minutes. Next, 15 mM iodoacetamide was added and the reaction was allowed to proceed for 45 minutes in the dark. Each sample was then buffer-exchanged into 10 mM TRIS, pH 7.5 using spin filters. 5 µg of trypsin was added to the reduced/alkylated sample and incubated for 4 hours at 37 °C. The trypsinized samples were divided into two equal parts. The first sample was deglycosylated by adding 1 µL PNGaseF (ProZyme) and 5 uL 10x digestion buffer and incubating overnight at room temperature, while the second sample was left untreated.

To determine the glycan occupancy, an aliquot of PNGase F treated and untreated tryptic digest was injected onto an Agilent 1290 UPLC coupled to an Agilent 5210 QTOF Mass Spectrometer for peptide mapping and mass alignment. Briefly, 5 µg TCR digest was injected on to a Waters 150mm x 2.1mm 1.7 µm C18 CSH UPLC column and gradient separated from 2% B ( 0.1% formic acid in acetonitrile) to 35% B over 30 minutes. The eluting peptides were injected into the mass spectrometer for mass analysis with a scan range from 200 to 2000 m/z, source temperature of 350 °C, capillary voltage of 4 kV, fragmentor at 250 V, capillary exit at 75 V, source gas at 40 PSI, and cone gas at 12 PSI. The peptide mass spectra was aligned to the protein sequence by Mass Hunter/Bioconfirm 7.0 software with a mass accuracy of 5 ppm resulting in over 90% sequence coverage. To measure glycan occupancy, deamidation of Asn was added as a variable modification and each predicted glycan was checked manually by extracted ion chromatogram (EIC) with 5 ppm mass accuracy. The ratio of Asn to Asp was calculated using the EIC and reported as percent glycan occupancy: Asn being unoccupied and Asp being occupied since the enzymatic deglycosylation converts Asn to Asp.

### Hydrogen/Deuterium Exchange coupled with mass spectrometry (HDX-MS).

HDX-MS experiments were performed on a Waters nanoACQUITY system with HDX technology (Wales, et al., Anal Chem, 2008. 80(17): 6815-20), including a LEAP HDX robotic liquid handling system. The deuterium exchange experiment was initiated by adding 55 µL of D₂O buffer containing 0.1x PBS to 5 µl of each protein (concentration was 25 µM) at 15 °C for various amounts of time (0s, 10s, 1 min, 10min, 60min, and 240 min). The reaction was quenched using equal volume of was 0.32 M TCEP, 0.1 M phosphate pH 2.5 for two minutes at 1 °C. 50 µL of the quenched reaction was injected on to an on-line pepsin column (Waters BEH Enzymate) at 14°C, using 0.2% formic acid in water as the mobile phase at a flow rate of 100 µL/ min for 4 min. The resulting peptic peptides were then separated on a C18 column (Waters, Acquity UPLC BEH C18, 1.7 µm, 1.0 mm × 50 mm) fit with a Vanguard trap column using a 3 to 85% acetonitrile (containing 0.2% formic acid) gradient over 10 min at a flow rate of 50 µL/min. The separated peptides were directed into a Waters Xevo G2 time-of-flight (qTOF) mass spectrometer. The mass spectrometer was set to collect data in the MS^{E}, ESI⁺ mode; in a mass acquisition range of *m*/*z* 255.00-1950.00; with a scan time of 0.5 s. The Xevo G2 was calibrated with Glu-fibrinopeptide prior to use. All acquired data was mass corrected using a 2 µg/ml solution of LeuEnk in 50% ACN, 50% H₂O and 0.1% FA at a flowrate of 5 µl/min every 30 s (*m*/*z* of 556.2771). The peptides were identified by Waters Protein Lynx Global Server 3.02. The processing parameters were set to low energy threshold at 100.0 counts, an elevated energy threshold at 50.0 counts and an intensity threshold at 1500.0 counts. The resulting peptide list was imported to Waters DynamX 3.0 software, with threshold of 5 ppm mass, 20% fragments ions per peptide based on peptide length. The relative deuterium incorporation for each peptide was determined by processing the MS data for deuterated samples along with the non-deuterated control in DynamX 3.0 (Waters Corporation).

### Pharmacokinetic measurements.

Pharmacokinetics measurements of the TCR/CD3 IgG-like BsAbs were performed as described previously (Lewis, et al., Nat Biotechnol, 2014. 32(2): 191-8). To evaluate whether anti-drug antibodies were being generated in the mice against the BsAbs, ELISAs were carried out by coating a High Bind U-well 96-well plate (Greiner Microlon) with 2 µg/mL BsAb in 50 mM sodium carbonate, pH 9.3 overnight at 4 °C. The plates were washed with PBS+0.1% Tween20 (PBST), blocked for 1 hour with Blocker^{™} Casein in PBS (Thermo Scientific) at room temperature (RT), and washed with PBST. Mouse plasma dilutions starting at 1:50 with 1:3 serial dilutions in Blocker^{™} Casein in PBS were added to the plate for 1 hour at room temperature. The plates were washed with PBST and adding a 1:1000 goat anti-mouse-kappa-AP polyclonal (Southern Biotech Cat#1050-04):Blocker ^{™} Casein primary antibody was added and incubated at 1 hour. The detection reagent was washed off followed by the addition of PNPP Substrate (Thermo Scientific). Colorimetric readout was performed by reading the absorbance at 450 nm on a SpectraMax 190 plate reader.

### T cell redirected lysis assays.

Saos-2 cells were from ATCC and the 624.38 cells were from the NCI/NIH DTP,DCTD Tumor Repository and both were cultured in Complete Media (RPMI 1640, Corning). Primary naive T cells were from ALLCELLS. For the assay, the tumor cells were removed from their culture flask using Accutase and spun down for 10 min at 1200 RPM. The tumor cells were re-suspended in Complete Media and seeded at 5000 cells/well in 96-well black, clear bottom plates (Perkin Elmer) and incubated for 16-24 hours in a 5% CO₂ incubator. The cells were then pulsed with 6 µg/mL SLLMWITQC (NY-ESO-1) peptide (synthesized by CPC Scientific), 50 µL total volume, for 3 hours. TCR/CD3 bifunctionals were then titrated onto the cells using 10X dilutions starting at 100 nM and ending at 0.001 nM in triplicate (50 µL/sample) for 30 minutes. During this period, primary T cells were thawed and washed twice in Complete Media and gentamicin. T cells (100 µL) were then added at 50K cells/well (200 mL total volume). Cells were incubated for 48 hours at 5% CO₂ and 37 °C. At 48 hours, the plates were washed gently (twice) with serum free RPMI 1640. Next 100 µL RPMI 1640 and 100 µL Cell Titer Glo reagent (Promega) were added, mixed for two minutes on a shaker (slowly) and incubated for 10 minutes in the dark. Lastly, the luminescence was read on an EnVision 2130 multilabel reader.

### Flow cytometry.

Cell culture was performed as described above. The day before running flow cytometry, T25 flasks were seeded with Jurkat, Saos-2 or 624.38 cells in Growth buffer (RPMI/10% fetal bovine serum (FBS)/gentamicin - Gibco). For the tumor cells, the next morning, cells were washed once with Growth buffer. Peptide was added to the tumor cells (or not as a negative control) at 6 µg/mL for 3 hours at 37 °C, 5% CO₂. Next, excess peptide was aspirated off and the cells were washed 3X with PBS buffer. All subsequent steps were peformed on ice. The tumor cells were lifted from the T25 flasks using Accutase (Innovative Technologies). The Jurkat cells grow in suspension. Cells were transferred to centrifuge tubes and pelleted by centrifugation at 1200 RPM for 7 minutes. Henceforth, 'Wash buffer' was PBS/2% FBS/0.05% NaN₃. 'Blocking buffer' was Wash buffer supplemented with 10% Normal Goat Serum/10% FBS/Human BD Fc Block (BD). The cells were resuspended in Block buffer for 15 minutes, pelleted, Washed 3X, and resuspended in Block buffer before adding 50 µL of the cells (0.2 x 10⁶) to 96-well plates (Corning 3799). The TCR/CD3 bifunctionals and control mAbs were added to the wells at 30, 3, 0.3, and 0.03 µg/mL and incubated 45 minutes. The cells were pelleted, washed, and the supernatants were aspirated again before adding 100 µL diluted R-Phycoerythrin-conjugated AffiniPure Goat Anti-Human IgG-Fc (Jackson) or Anti-Human Lambda LC (Jackson) in Block buffer for 45 minutes. The cells were pelleted and washed again. Finally, the cells were resuspended in Block buffer with 1: 1000 PI (Molecular Probes) and covered with foil. The cells were then sorted on a Fortessa H647780000006 flow cytometer acquiring with Diva software (BD) and data was analyzed using FloJo version 10.0.08.

### Data availability

The coordinates for the TCR constant domain structure, and the corresponding structure factors, have been deposited in the Protein Data Bank (http://www.rcsb.org) under accession code 6U07.

### Sequence Listing

**SEQ ID NO.1** WT TCRα constant domain (Cα) 
PYIQNPDPAVYQLRDSKSSDKSVCLFTDFDSQTNVSQSKDSDVYITDKTVLDMRSMDFKSNS
AVAWSNKSDFACANAFNNSIIPEDTFFPSPESSC
**SEQ ID NO. 2** WT TCRβ constant domain (Cβ) 
EDLKNVFPPEVAVFEPSEAEISHTQKATLVCLATGFYPDHVELSWWVNGKEVHSGVSTDPQP
LKEQPALNDSRYALSSRLRVSATFWQDPRNHFRCQVQFYGLSENDEWTQDRAKPVTQIVSAE
AWGRADC
**SEQ ID NO.3** WT TCRα constant domain (Cα) with disulfide mutation 
PYIQNPDPAVYQLRDSKSSDKSVCLFTDFDSQTNVSQSKDSDVYITDKCVLDMRSMDFKSNS
AVAWSNKSDFACANAFNNSIIPEDTFFPSPESSC
**SEQ ID NO. 4** WT TCRβ constant domain (Cβ) with disulfide mutation 
EDLKNVFPPEVAVFEPSEAEISHTQKATLVCLATGFYPDHVELSWWVNGKEVHSGVCTDPQP
LKEQPALNDSRYALSSRLRVSATFWQDPRNHFRCQVQFYGLSENDEWTQDRAKPVTQIVSAE
AWGRADC
**SEQ ID NO.5** Cα for TCR with 3 stabilizing mutations minus disulfide
   Alpha constant domain minus disulfide_S139F_T150I_A190T 
PYIQNPDPAVYQLRDSKSSDKFVCLFTDFDSQINVSQSKDSDVYITDKTVLDMRSMDFKSNS
AVAWSNKSDFTCANAFNNSIIPEDTFFPSPESSC
**SEQ ID NO.6** Cα for TCR with 3 stabilizing mutations with disulfide mutation
   Alpha constant domain with disulfide_S139F_T150I_A190T 
PYIQNPDPAVYQLRDSKSSDKFVCLFTDFDSQINVSQSKDSDVYITDKCVLDMRSMDFKSNS
AVAWSNKSDFTCANAFNNSIIPEDTFFPSPESSC
**SEQ ID NO. 7** Cβ for TCR with 4 stabilizing mutations minus disulfide
   Beta constant E134K_H139R_D155P_S170D 
EDLKNVFPPEVAVFEPSKAEISRTQKATLVCLATGFYPPHVELSWWVNGKEVHDGVSTDPQP
LKEQPALNDSRYALSSRLRVSATFWQDPRNHFRCQVQFYGLSENDEWTQDRAKPVTQIVSAE
AWGRADC
**SEQ ID NO. 8** Cβ for TCR with 4 stabilizing mutations with disulfide mutation
   Beta constant E134K_H139R_D155P_S170D 
EDLKNVFPPEVAVFEPSKAEISRTQKATLVCLATGFYPPHVELSWWVNGKEVHDGVCTDPQP
LKEQPALNDSRYALSSRLRVSATFWQDPRNHFRCQVQFYGLSENDEWTQDRAKPVTQIVSAE
AWGRADC
**SEQ ID NO.9** NY-ESO-1 Cα WT without disulfide 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPLLDGTYIPTFGRGTSLIVHPYIQNPDPAVYQ
LRDSKSSDKSVCLFTDFDSQTNVSQSKDSDVYITDKTVLDMRSMDFKSNSAVAWSNKSDFTC
ANAFNNSIIPEDTFFPSPESSC
**SEQ ID NO.10** NY-ESO-1 Cα WT with disulfide 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPLLDGTYIPTFGRGTSLIVHPYIQNPDPAVYQ
LRDSKSSDKSVCLFTDFDSQTNVSQSKDSDVYITDKCVLDMRSMDFKSNSAVAWSNKSDFTC
ANAFNNSIIPEDTFFPSPESSC
**SEQ ID NO.11** NY-ESO-1 Cα with disulfide_S139F_T150I_A190T 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPLLDGTYIPTFGRGTSLIVHPYIQNPDPAVYQ
LRDSKSSDKFVCLFTDFDSQINVSQSKDSDVYITDKCVLDMRSMDFKSNSAVAWSNKSDFTC
ANAFNNSIIPEDTFFPSPESSC
**SEQ ID NO.12** NY-ESO-1 Cα without disulfide_S139F_T150I A190T 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPLLDGTYIPTFGRGTSLIVHPYIQNPDPAVYQ
LRDSKSSDKFVCLFTDFDSQINVSQSKDSDVYITDKTVLDMRSMDFKSNSAVAWSNKSDFTC
ANAFNNSIIPEDTFFPSPESSC
**SEQ ID NO.13** NY-ESO-1 Cβ WT without disulfide 
GVTQTPKFQVLKTGQSMTLQCAQDMNHEYMSWYRQDPGMGLRLIHYSVAIQTTDQGEVPNGY
NVSRSTIEDFPLRLLSAAPSQTSVYFCASSYVGDTGELFFGEGSRLTVLEDLKNVFPPEVAV
FEPSEAEISHTQKATLVCLATGFYPDHVELSWWVNGKEVHSGVSTDPQPLKEQPALNDSRYA
LSSRLRVSATFWQDPRNHFRCQVQFYGLSENDEWTQDRAKPVTQIVSAEAWGRADC
**SEQ ID NO.14** NY-ESO-1 Cβ WT with disulfide 
GVTQTPKFQVLKTGQSMTLQCAQDMNHEYMSWYRQDPGMGLRLIHYSVAIQTTDQGEVPNGY
NVSRSTIEDFPLRLLSAAPSQTSVYFCASSYVGDTGELFFGEGSRLTVLEDLKNVFPPEVAV
FEPSEAEISHTQKATLVCLATGFYPDHVELSWWVNGKEVHSGVCTDPQPLKEQPALNDSRYA
LSSRLRVSATFWQDPRNHFRCQVQFYGLSENDEWTQDRAKPVTQIVSAEAWGRADC
**SEQ ID NO.15** NY-ESO-1 Cβ with disulfide_H139R_D155P_S170D 
GVTQTPKFQVLKTGQSMTLQCAQDMNHEYMSWYRQDPGMGLRLIHYSVAIQTTDQGEVPNGY
NVSRSTIEDFPLRLLSAAPSQTSVYFCASSYVGDTGELFFGEGSRLTVLEDLKNVFPPEVAV
FEPSKAEISRTQKATLVCLATGFYPPHVELSWWVNGKEVHDGVCTDPQPLKEQPALNDSRYA
LSSRLRVSATFWQDPRNHFRCQVQFYGLSENDEWTQDRAKPVTQIVSAEAWGRADC
**SEQ ID NO.16** NY-ESO-1 Cβ without disulfide_H139R_D155P_S170D 
GVTQTPKFQVLKTGQSMTLQCAQDMNHEYMSWYRQDPGMGLRLIHYSVAIQTTDQGEVPNGY
NVSRSTIEDFPLRLLSAAPSQTSVYFCASSYVGDTGELFFGEGSRLTVLEDLKNVFPPEVAV
FEPSKAEISRTQKATLVCLATGFYPPHVELSWWVNGKEVHDGVSTDPQPLKEQPALNDSRYA
LSSRLRVSATFWQDPRNHFRCQVQFYGLSENDEWTQDRAKPVTQIVSAEAWGRADC
**SEQ ID NO.17** CE10 Cα WT with disulfide 
QQVKQNSPSLSVQEGRISILNCDYTNSMFDYFLWYKKYPAEGPTFLISISSIKDKNEDGRFT
VFLNKSAKHLSLHIVPSQPGDSAVYFCAASGDFNKFYFGSGTKLNVKPYIQNPDPAVYQLRD
SKSSDKSVCLFTDFDSQTNVSQSKDSDVYITDKCVLDMRSMDFKSNSAVAWSNKSDFACANA
FNNSIIPEDTFFPSPESSC
**SEQ ID NO.18** CE10 Cα with disulfide_S139F_T150I_A190T 
QQVKQNSPSLSVQEGRISILNCDYTNSMFDYFLWYKKYPAEGPTFLISISSIKDKNEDGRFT
VFLNKSAKHLSLHIVPSQPGDSAVYFCAASGDFNKFYFGSGTKLNVKPYIQNPDPAVYQLRD
SKSSDKFVCLFTDFDSQINVSQSKDSDVYITDKCVLDMRSMDFKSNSAVAWSNKSDFTCANA
FNNSIIPEDTFFPSPESSC
**SEQ ID NO.19** CE10 Cβ WT with disulfide 
EAGVAQSPRYKIIEKRQSVAFWCNPISGHATLYWYQQILGQGPKLLIQFQNNGVVDDSQLPK
DRFSAERLKGVDSTLKIQPAKLEDSAVYLCASSLGTGLGEQYFGPGTRLTVTEDLKNVFPPE
VAVFEPSEAEISHTQKATLVCLATGFYPDHVELSWWVNGKEVHSGVCTDPQPLKEQPALNDS
RYALSSRLRVSATFWQDPRNHFRCQVQFYGLSENDEWTQDRAKPVTQIVSAEAWGRADC
**SEQ ID NO.20** CE10 Cβ with disulfide_E134K_H139R_D155P_S170D 
EAGVAQSPRYKIIEKRQSVAFWCNPISGHATLYWYQQILGQGPKLLIQFQNNGVVDDSQLPK
DRFSAERLKGVDSTLKIQPAKLEDSAVYLCASSLGTGLGEQYFGPGTRLTVTEDLKNVFPPE
VAVFEPSKAEISRTQKATLVCLATGFYPPHVELSWWVNGKEVHDGVCTDPQPLKEQPALNDS
RYALSSRLRVSATFWQDPRNHFRCQVQFYGLSENDEWTQDRAKPVTQIVSAEAWGRADC
**SEQ ID NO.21** anti NEF134-10 HIV TCR Cα WT with disulfide 
QQKEVEQNSGPLSVPEGAIASLNCTYSDRGSQSFFWYRQYSGKSPELIMSIYSNGDKEDGRF
TAQLNKASQYVSLLIRDSQPSDSATYLWGTYNQGGKLIFGQGTELSVKPYIQNPDPAVYQLR
DSKSSDKSVCLFTDFDSQTNVSQSKDSDVYITDKCVLDMRSMDFKSNSAVAWSNKSDFACAN
AFNNSIIPEDTFFPSPESSC
**SEQ ID NO.22** anti NEF134-10 HIV TCR Cα with disulfide_S139F_T150I_ A190T 
QQKEVEQNSGPLSVPEGAIASLNCTYSDRGSQSFFWYRQYSGKSPELIMSIYSNGDKEDGRF
TAQLNKASQYVSLLIRDSQPSDSATYLWGTYNQGGKLIFGQGTELSVKPYIQNPDPAVYQLR
DSKSSDKFVCLFTDFDSQINVSQSKDSDVYITDKCVLDMRSMDFKSNSAVAWSNKSDFTCAN
AFNNSIIPEDTFFPSPESSC
**SEQ ID NO.23** anti NEF134-10 HIV TCR Cβ WT with disulfide 
QVTQNPRYLITVTGKKLTVTCSQNMNHEYMSWYRQDPGLGLRQIYYSMNVEVTDKGDVPEGY
KVSRKEKRNFPLILESPSPNQTSLYFCASSGASHEQYFGPGTRLTVTEDLKNVFPPEVAVFE
PSEAEISHTQKATLVCLATGFYPDHVELSWWVNGKEVHSGVCTDPQPLKEQPALNDSRYALS
SRLRVSATFWQDPRNHFRCQVQFYGLSENDEWTQDRAKPVTQIVSAEAWGRADC
**SEQ ID NO.24** anti NEF134-10 HIV TCR Cβ with disulfide_ E134K_H139R_D155P_S170D 
QVTQNPRYLITVTGKKLTVTCSQNMNHEYMSWYRQDPGLGLRQIYYSMNVEVTDKGDVPEGY
KVSRKEKRNFPLILESPSPNQTSLYFCASSGASHEQYFGPGTRLTVTEDLKNVFPPEVAVFE
PSKAEISRTQKATLVCLATGFYPPHVELSWWVNGKEVHDGVCTDPQPLKEQPALNDSRYALS
SRLRVSATFWQDPRNHFRCQVQFYGLSENDEWTQDRAKPVTQIVSAEAWGRADC
**SEQ ID NO.25** MAGE-A3 Cα WT with disulfide 
KQEVTQIPAALSVPEGENLVLNCSFTDSAIYNLQWFRQDPGKGLTSLLYVRPYQREQTSGRL
NASLDKSSGRSTLYIAASQPGDSATYLCAVRPGGAGPFFVVFGKGTKLSVIPYIQNPDPAVY
QLRDSKSSDKSVCLFTDFDSQTNVSQSKDSDVYITDKCVLDMRSMDFKSNSAVAWSNKSDFA
CANAFNNSIIPEDTFFPSPESSC
**SEQ ID NO.26** MAGE-A3 Cα with disulfide_S139F_T150I_A190T 
KQEVTQIPAALSVPEGENLVLNCSFTDSAIYNLQWFRQDPGKGLTSLLYVRPYQREQTSGRL
NASLDKSSGRSTLYIAASQPGDSATYLCAVRPGGAGPFFVVFGKGTKLSVIPYIQNPDPAVY
QLRDSKSSDKFVCLFTDFDSQINVSQSKDSDVYITDKCVLDMRSMDFKSNSAVAWSNKSDFT
CANAFNNSIIPEDTFFPSPESSC
**SEQ ID NO.27** MAGE-A3 Cβ WT with disulfide 
GVTQTPRYLIKTRGQQVTLSCSPISGHRSVSWYQQTPGQGLQFLFEYFSETQRNKGNFPGRF
SGRQFSNSRSEMNVSTLELGDSALYLCASSFNMATGQYFGPGTRLTVTEDLKNVFPPEVAVF
EPSEAEISHTQKATLVCLATGFYPDHVELSWWVNGKEVHSGVCTDPQPLKEQPALNDSRYAL
SSRLRVSATFWQDPRNHFRCQVQFYGLSENDEWTQDRAKPVTQIVSAEAWGRADC
**SEQ ID NO.28** MAGE-A3 Cβ with disulfide_E134K_H139R_D155P_S170D 
GVTQTPRYLIKTRGQQVTLSCSPISGHRSVSWYQQTPGQGLQFLFEYFSETQRNKGNFPGRF
SGRQFSNSRSEMNVSTLELGDSALYLCASSFNMATGQYFGPGTRLTVTEDLKNVFPPEVAVF
EPSKAEISRTQKATLVCLATGFYPPHVELSWWVNGKEVHDGVCTDPQPLKEQPALNDSRYAL
SSRLRVSATFWQDPRNHFRCQVQFYGLSENDEWTQDRAKPVTQIVSAEAWGRADC
**SEQ ID NO.29** IgG1AA _N297Q NY-ESO-1 WT Cα with disulfide Fc 7.8.60A 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPLLDGTYIPTFGRGTSLIVHPYIQNPDPAVYQ
LRDSKSSDKSVCLFTDFDSQTNVSQSKDSDVYITDKCVLDMRSMDFKSNSAVAWSNKSDFAC
ANAFNNSIIPEDTFFPSPESSCDKTHTCPPCPAPEAAGGPSVFLFPPKPKDTLMISRTPEVT
CVVVDVSHEDPEVKFNWYVDGVEVHNAKTKPREEQYQSTYRVVSVLTVLHQDWLNGKEYKCK
VSNKALPAPIEKTISKAKGQPREPQVYTLPPSREEMTDNQVSLTCLVKGFYPSDIAVEWESN
GQPENNYKTTPPVLMSDGSFFLASKLTVDKSRWQQGNVFSCSVMHEALHNHYTQKSLSLSPG
K
**SEQ ID NO.30** IgG1AA _N297Q NY-ESO-1 Cα _S139F_T150I_A190T with disulfide and CH3 7.8.60A heterodimer mutations 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPLLDGTYIPTFGRGTSLIVHPYIQNPDPAVYQ
 
LRDSKSSDKFVCLFTDFDSQINVSQSKDSDVYITDKCVLDMRSMDFKSNSAVAWSNKSDFTC
ANAFNNSIIPEDTFFPSPEPKSCDKTHTCPPCPAPEAAGGPSVFLFPPKPKDTLMISRTPEV
TCVVVDVSHEDPEVKFNWYVDGVEVHNAKTKPREEQYQSTYRVVSVLTVLHQDWLNGKEYKC
KVSNKALPAPIEKTISKAKGQPREPQVYTLPPSREEMTDNQVSLTCLVKGFYPSDIAVEWES
NGQPENNYKTTPPVLMSDGSFFLASKLTVDKSRWQQGNVFSCSVMHEALHNHYTQKSLSLSP
GK
**SEQ ID NO.31** IgG1AA_N297Q SP34 mVH with 7.8.60B heterodimer mutations 
EVQLVESGGGLVQPKGSLKLSCAASGFTFNTYAMNWVRQAPGKGLEWVARIRSKYNNYATYY
ADSVKDRFTISRDDSQSLLYLQMNNLKTEDTAMYYCVRHGNFGNSYVSWFAYWGQGTLVTVS
AASTKGPSVFPLAPSSKSTSGGTAALGCLVADYFPEPVTVSWNSGALTSGVHTFPAVLQSSG
LYSLASVVTVPSSSLGTQTYICNVNHKPSNTKVDKRVEPKSCDKTHTCPPCPAPEAAGGPSV
FLFPPKPKDTLMISRTPEVTCVVVDVSHEDPEVKFNWYVDGVEVHNAKTKPREEQYQSTYRV
VSVLTVLHQDWLNGKEYKCKVSNKALPAPIEKTISKAKGQPRRPRVYTLPPSREEMTKNQVS
LVCLVKGFYPSDIAVEWESNGQPENNYKTTPPVLDSDGSFFLYSVLTVDKSRWQQGNVFSCS
VMHEALHNHYTQKSLSLSPGK
**SEQ ID NO.32** Ch SP34 mVL Lambda 
QAVVTQESALTTSPGETVTLTCRSSTGAVTTSNYANWVQEKPDHLFTGLIGGTNKRAPGVPA
RFSGSLIGDKAALTITGAQTEDEAIYFCALWYSNLWVFGGGTKLTVLGQPKAAPSVTLFPPS
SEELQANKATLVCLISDFYPGAVTVAWKADSSPVKAGVETTTPSKQSNNKYAASSYLSLTPE
QWKSHRSYSCQVTHEGSTVEKTVAPTEC
**SEQ ID NO.33** NY-ESO-1 Cα _S139F_T150I_A190T with disulfide_(G4S)4 linker_Ch SP34 mVH tandem FAb 
HHHHHHHHGSQEVTQIPAALSVPEGENLVLNCSFTDSAIYNLQWFRQDPGKGLTSLLLISPW
QREQTSGRLNASLDKSSGRSTLYIAASQPGDSATYLCAVRPLLDGTYIPTFGRGTSLIVHPY
IQNPDPAVYQLRDSKSSDKFVCLFTDFDSQINVSQSKDSDVYITDKCVLDMRSMDFKSNSAV
AWSNKSDFTCANAFNNSIIPEDTFFPSPESSCGGGGSGGGGSGGGGSGGGGSEVQLVESGGG
LVQPKGSLKLSCAASGFTFNTYAMNWVRQAPGKGLEWVARIRSKYNNYATYYADSVKDRFTI
SRDDSQSLLYLQMNNLKTEDTAMYYCVRHGNFGNSYVSWFAYWGQGTLVTVSAASTKGPSVF
PLAPCSRSTSESTAALGCLVKDYFPEPVTVSWNSGALTSGVHTFPAVLQSSGLYSLSSVVTV
PSSSLGTKTYTCNVDHKPSNTKVDKRVESKYG
**SEQ ID NO.34** IgG1AA_N297Q NY-ESO-1 Cα _S139F_T150I_A190T Deglycosylated with disulfide 7.8.60A 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPLLDGTYIPTFGRGTSLIVHPYIQNPDPAVYQ
LRDSKSSDKFVCLFTDFDSQIQVSQSKDSDVYITDKCVLDMRSMDFKSNSAVAWSNKADFTC
ANAFQNSIIPEDTFFPSPEPKSCDKTHTCPPCPAPEAAGGPSVFLFPPKPKDTLMISRTPEV
TCVVVDVSHEDPEVKFNWYVDGVEVHNAKTKPREEQYQSTYRVVSVLTVLHQDWLNGKEYKC
KVSNKALPAPIEKTISKAKGQPREPQVYTLPPSREEMTDNQVSLTCLVKGFYPSDIAVEWES
NGQPENNYKTTPPVLMSDGSFFLASKLTVDKSRWQQGNVFSCSVMHEALHNHYTQKSLSLSP
GK
**SEQ ID NO.35** IgG1AA_N297Q NY-ESO-1 #107 Cα _S139F_T150I_A190T with disulfide 7.8.60A 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPFTGGGYIPTFGRGTSLIVHPYIQNPDPAVYQ
LRDSKSSDKFVCLFTDFDSQINVSQSKDSDVYITDKCVLDMRSMDFKSNSAVAWSNKSDFTC
ANAFNNSIIPEDTFFPSPEPKSCDKTHTCPPCPAPEAAGGPSVFLFPPKPKDTLMISRTPEV
TCVVVDVSHEDPEVKFNWYVDGVEVHNAKTKPREEQYQSTYRVVSVLTVLHQDWLNGKEYKC
KVSNKALPAPIEKTISKAKGQPREPQVYTLPPSREEMTDNQVSLTCLVKGFYPSDIAVEWES
NGQPENNYKTTPPVLMSDGSFFLASKLTVDKSRWQQGNVFSCSVMHEALHNHYTQKSLSLSP
GK
**SEQ ID NO. 36** NY-ESO-1#107 Cβ with disulfide_E134K_H139R _D155P_S170D 
GVTQTPKFQVLKTGQSMTLQCAQDMNHEYMSWYRQDPGMGLRLIHYSVAIQTTDQGEVPNGY
NVSRSTTEDFPLRLLSAAPSQTSVYFCASSYVGNTGELFFGEGSRLTVLEDLKNVFPPEVAV
FEPSKAEISRTQKATLVCLATGFYPPHVELSWWVNGKEVHDGVCTDPQPLKEQPALNDSRYA
LSSRLRVSATFWQDPRNHFRCQVQFYGLSENDEWTQDRAKPVTQIVSAEAWGRADC
**SEQ ID NO.37** IgG1AA_N297Q NY-ESO-1 #113 Cα _S139F_T150I_A190T with disulfide 7.8.60A 
QEVTQIPAALSVPEGENLVLNCSFTDSAIYNLQWFRQDPGKGLTSLLLITPWQREQTSGRLN
ASLDKSSGRSTLYIAASQPGDSATYLCAVRPLLDGTYIPTFGRGTSLIVHPYIQNPDPAVYQ
LRDSKSSDKFVCLFTDFDSQINVSQSKDSDVYITDKCVLDMRSMDFKSNSAVAWSNKSDFTC
ANAFNNSIIPEDTFFPSPEPKSCDKTHTCPPCPAPEAAGGPSVFLFPPKPKDTLMISRTPEV
TCVVVDVSHEDPEVKFNWYVDGVEVHNAKTKPREEQYQSTYRVVSVLTVLHQDWLNGKEYKC
KVSNKALPAPIEKTISKAKGQPREPQVYTLPPSREEMTDNQVSLTCLVKGFYPSDIAVEWES
NGQPENNYKTTPPVLMSDGSFFLASKLTVDKSRWQQGNVFSCSVMHEALHNHYTQKSLSLSP
GK
**SEQ ID NO.38** NY-ESO-1#113 Cβ with disulfide_E134K_H139R_D155P_S170D 
GVTQTPKFQVLKTGQSMTLQCAQDMNHEYMSWYRQDPGMGLRLIHYSVAIQTTDRGEVPNGY
NVSRSTIEDFPLRLLSAAPSQTSVYFCASSYLGNTGELFFGEGSRLTVLEDLKNVFPPEVAV
FEPSKAEISRTQKATLVCLATGFYPPHVELSWWVNGKEVHDGVCTDPQPLKEQPALNDSRYA
LSSRLRVSATFWQDPRNHFRCQVQFYGLSENDEWTQDRAKPVTQIVSAEAWGRADC
**SEQ ID NO.39** IgG1AA _N297Q NY-ESO-1 Cα _S139F_T150I_A190T with disulfide_3XG4S_SP34mVH 7.8.60B 
QEVTQIPAALSVPEGENLVLNCSFTDSAIYNLQWFRQDPGKGLTSLLLISPWQREQTSGRLN
ASLDKSSGRSTLYIAASQPGDSATYLCAVRPLLDGTYIPTFGRGTSLIVHPYIQNPDAVYQ
LRDSKSSDKFVCLFTDFDSQINVSQSKDSDVYITDKCVLDMRSMDFKSNSAVAWSNKSDFTC
ANAFNNSIIPEDTFFPSPESSCGGGGSGGGGSGGGGSGSEVQLVESGGGLVQPKGSLKLSCA
ASGFTFNTYAMNWVRQAPGKGLEWVARIRSKYNNYATYYADSVKDRFTISRDDSQSLLYLQM
NNLKTEDTAMYYCVRHGNFGNSYVSWFAYWGQGTLVTVSAASTKGPSVFPLAPSSKSTSGGT
AALGCLVADYFPEPVTVSWNSGALTSGVHTFPAVLQSSGLYSLASVVTVPSSSLGTQTYICN
VNHKPSNTKVDKRVEPKSCDKTHTCPPCPAPEAAGGPSVFLFPPKPKDTLMISRTPEVTCVV
VDVSHEDPEVKFNWYVDGVEVHNAKTKPREEQYQSTYRVVSVLTVLHQDWLNGKEYKCKVSN
KALPAPIEKTISKAKGQPRRPRVYTLPPSREEMTKNQVSLVCLVKGFYPSDIAVEWESNGQP
ENNYKTTPPVLDSDGSFFLYSVLTVDKSRWQQGNVFSCSVMHEALHNHYTQKSLSLSPGK
**SEQ ID NO.40** NY-ESO-1 Cα _S139F_T150I_A190T with disulfide_5XG4S_Ch SP34 mVH tandem FAb 
HHHHHHHHGSQEVTQIPAALSVPEGENLVLNCSFTDSAIYNLQWFRQDPGKGLTSLLLISPW
QREQTSGRLNASLDKSSGRSTLYIAASQPGDSATYLCAVRPLLDGTYIPTFGRGTSLIVHPY
IQNPDPAVYQLRDSKSSDKFVCLFTDFDSQINVSQSKDSDVYITDKCVLDMRSMDFKSNSAV
AWSNKSDFTCANAFNNSIIPEDTFFPSPESSCGGGGSGGGGSGGGGSGGGGSGGGSEVQLVE
SGGGLVQPKGSLKLSCAASGFTFNTYAMNWVRQAPGKGLEWVARIRSKYNNYATYYADSVKD
RFTISRDDSQSLLYLQMNNLKTEDTAMYYCVRHGNFGNSYVSWFAYWGQGTLVTVSAASTKG
PSVFPLAPCSRSTSESTAALGCLVKDYFPEPVTVSWNSGALTSGVHTFPAVLQSSGLYSLSS
VVTVPSSSLGTKTYTCNVDHKPSNTKVDKRVESKYG
**SEQ ID NO.41** (G4S)₃ linker
   GGGGSGGGGSGGGGS
**SEQ ID NO.42** (G4Q)₃ linker
   GGGGQGGGGQGGGGQ
**SEQ ID NO.43** (G4S)₄ linker
   GGGGSGGGGSGGGGSGGGGS
**SEQ ID NO.44** (G4S)₅ linker
   GGGGSGGGGSGGGGSGGGGSGGGGS
**SEQ ID NO.45** (G4Q)₄ linker
   GGGGQGGGGQGGGGQGGGGQ
**SEQ ID NO.46** (G4Q)₅ linker
   GGGGQGGGGQGGGGQGGGGQGGGGQ
**SEQ ID NO. 47** IgG1 hinge region
   EPKSCDKTHTCPPCP
**SEQ ID NO. 48** IgG1 Fc region with L234A_L235A_N297Q 
APEAAGGPSVFLFPPKPKDTLMISRTPEVTCVVVDVSHEDPEVKFNWYVDGVEVHNAKTKPR
EEQYQSTYRVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSKLTVDKSR
WQQGNVFSCSVMHEALHNHYTQKSLSLSPGK
**SEQ ID NO. 49** IgG4 (StoP) hinge region
   ESKYGPPCPPCP
**SEQ ID NO. 50** IgG4AA Fc region 
APEAAGGPSVFLFPPKPKDTLMISRTPEVTCVVVDVSQEDPEVQFNWYVDGVEVHNAKTKPR
EEQFNSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTVDKSR
WQEGNVFSCSVMHEALHNHYTQKSLSLSLG
**SEQ ID NO: 51** Human HPB-MLT Cβ 
EDLKNVFPPEVAVFEPSEAEISHTQKATLVCLATGFYPDHVELSWWVNGKEVHSGVSTDPQP
LKEQPALNDSRYCLSSRLRVSATFWQNPRNHFRCQVQFYGLSENDEWTQDRAKPVTQIVSAE
AWGRADC
**SEQ ID NO: 52** Human HPB-MLT Cα

NIQNPDPAVYQLRDSKSSDKSVCLFTDFDSQTNVSQSKDSDVYITDKTVLDMRSMDFKSNSA
VAWSNKSDFACANAFNNSIIPEDTFFPSPESSC

## Claims

1. A protein comprising:
a first polypeptide comprising a T cell receptor (TCR) alpha constant domain (Cα) comprising at least one of the following residues: phenylalanine at position 139, isoleucine at position 150, threonine at position 190 (residues numbered according to Kabat numbering); and/or
a second polypeptide comprising a TCR beta constant domain (Cβ) comprising at least one of the following residues: arginine at position 139, proline at position 155, aspartic acid or glutamic acid at position 170 (residues numbered according to Kabat numbering),
wherein the protein has a higher unfolding temperature (Tm) compared to a protein comprising the same amino acid sequence except that:
the Cα domain comprises serine at position 139, threonine at position 150, and alanine at position 190 (residues numbered according to Kabat numbering); and/or
the Cβ domain comprises glutamic acid at position 134, histidine at position 139, aspartic acid at position 155, and serine at position 170 (residues numbered according to Kabat numbering).

2. The protein of claim 1, wherein:
(a) the first polypeptide comprises a Cα comprising at least one of the following residues: phenylalanine at position 139, isoleucine at position 150, threonine at position 190 (residues numbered according to Kabat numbering); and the second polypeptide comprises a Cβ comprising at least one of the following residues: lysine at position 134, arginine at position 139, proline at position 155, aspartic acid or glutamic acid at position 170 (residues numbered according to Kabat numbering);
(b) the first polypeptide comprises a Cα domain comprising the following residues: phenylalanine at position 139, isoleucine at position 150, threonine at position 190 (residues numbered according to Kabat numbering); and the second polypeptide comprises a Cβ domain comprising the following residues: lysine at position 134, arginine at position 139, proline at position 155, aspartic acid at position 170 (residues numbered according to Kabat numbering); or
(c) the first polypeptide comprises a Cα domain comprising the following residues: phenylalanine at position 139 and threonine at position 190 (residues numbered according to Kabat numbering); and the second polypeptide comprises a Cβ domain comprising the following residues: lysine at position 134, arginine at position 139, proline at position 155, aspartic acid at position 170 (residues numbered according to Kabat numbering).

3. The protein of any claim 1 or 2, wherein:
(a) the first polypeptide is linked to the second polypeptide by an inter-chain disulfide bond; and/or
(b) the Cα domain further comprises a cysteine residue at position 166 (residue numbered according to Kabat numbering), and the Cβ domain further comprises a cysteine residue at position 173 (residue numbered according to Kabat numbering), wherein the first polypeptide and the second polypeptide are linked by an inter-chain disulfide bond between the cysteine residue at position 166 of Cα and the cysteine residue at position 173 of Cβ.

4. The protein of claim 2 or 3, wherein:
(a) the Cα domain comprises SEQ ID NO: 5 and the Cβ domain comprises SEQ ID NO: 7;
(b) the Cα domain consists of SEQ ID NO: 5 and the Cβ domain consists of SEQ ID NO: 7;
(c) the Cα domain comprises SEQ ID NO: 6 and the Cβ domain comprises SEQ ID NO: 8; or
(d) the Cα domain consists of SEQ ID NO: 6 and the Cβ domain consists of SEQ ID NO: 8.

5. The protein of any one of claims 1-4, wherein:
the first polypeptide further comprises a TCR alpha variable domain (Vα); and
the second polypeptide further comprises a TCR beta variable domain (Vβ),
wherein the Vα and Vβ form an antigen binding domain that binds an antigen.

6. The protein of claim 5, wherein:
(a) the Vα is fused to the N-terminus of Cα and the Vβ is fused to the N-terminus of Cβ; and/or
(b) the antigen is a tumor antigen or a viral antigen.

7. The protein of any one of claims 1-6, wherein the protein further comprises a second antigen binding domain.

8. The protein of claim 7, wherein:
(a) the second antigen binding domain binds an antigen on T cell surface; or
(b) the second antigen binding domain binds CD3.

9. The protein of claim 7 or 8, wherein:
(a) the second antigen binding domain is a scFv, Fab, Fab', (Fab')₂, single domain antibody, or camelid VHH domain; or
(b) the second antigen binding domain is a Fab.

10. The protein of claim 9, wherein the second antigen binding domain is a Fab which comprises a Fab heavy chain comprising a heavy chain variable domain (VH) and a human IgG CH1 domain, and a Fab light chain comprising a light chain variable domain (VL) and a human light chain constant domain (CL), wherein the VH and VL form the second antigen binding domain that binds CD3.

11. The protein of claim 9 or 10, wherein the second antigen binding domain is a Fab and the protein comprises three polypeptides:
a first polypeptide comprising Vα - Cα - linker- VH - CH1;
a second polypeptide comprising Vβ - Cβ; and
a third polypeptide comprising VL - CL;
wherein the second and third polypeptides are linked to the first polypeptide by inter-chain disulfide bonds.

12. The protein of any one of claims 1-11, wherein:
(a) the protein further comprises a human IgG Fc region (Fc); or
(b) the protein further comprises a human IgG Fc region that is a modified human IgG Fc region with reduced effector function compared to the corresponding wild type human IgG Fc region.

13. The protein of any one of claims 1-10, or 12, wherein the protein comprises four polypeptides:
a first polypeptide comprising Vα - Cα - hinge - first Fc region;
a second polypeptide comprising Vβ - Cβ;
a third polypeptide comprising VL - CL; and
a fourth polypeptide comprising VH - CH1 - hinge - second Fc region;
wherein the second and fourth polypeptides are linked to the first polypeptide by inter-chain disulfide bonds; and the third polypeptide is linked to the fourth polypeptide by inter-chain disulfide bonds.

14. The protein of claim 12, wherein the protein comprises four polypeptides:
a first polypeptide comprising Vα - Cα - linker - VH- CH1 - hinge - first Fc region;
a second polypeptide comprising Vβ - Cβ;
a third polypeptide comprising VL-CL;
a fourth polypeptide comprising a second Fc region; and
wherein the second, third, and fourth polypeptides are linked to the first polypeptide by inter-chain disulfide bonds.

15. The protein of claim 11 or 14, wherein the linker comprises an amino acid sequence selected from any one of SEQ ID NOs: 41-46.

16. The protein of claim 13 or 14, wherein:
(a) the hinge region comprises SEQ ID NO: 47 and the first and second Fc regions comprise SEQ ID NO: 48;
(b) the hinge region comprises SEQ ID NO: 49 and the first and second Fc regions comprise SEQ ID NO: 50;
(c) the first and second Fc regions comprise a set of CH3 heterodimerization mutations; or
(d) the first and second Fc regions comprise a set of CH3 heterodimerization mutations, wherein one of the first or second Fc region comprises a CH3 domain comprising an alanine at residue 407, a methionine at residue 399, and an aspartic acid at residue 360; and the other of said first or second Fc region comprises a CH3 domain comprising a valine at residue 366, a valine at residue 409, and an arginine at residues 345 and 347 (residues numbered according to the EU Index Numbering).

17. The protein of any one of claims 1-16, wherein the protein is a soluble protein.

18. The protein of any one of claims 1-17, wherein:
(a) the protein has increased stability compared to a protein comprising the same amino acid sequence except that: the Cα domain comprising serine at position 139, threonine at position 150, and alanine at position 190 (residues numbered according to Kabat numbering); and the Cβ domain comprising glutamic acid at position 134, histidine at position 139, aspartic acid at position 155, and serine at position 170 (residues numbered according to Kabat numbering);
(b) when expressed under the same condition, the protein has increased expression level compared to a protein comprising the same amino acid sequence except that: the Cα domain comprising serine at position 139, threonine at position 150, and alanine at position 190 (residues numbered according to Kabat numbering); and the Cβ domain comprising glutamic acid at position 134, histidine at position 139, aspartic acid at position 155, and serine at position 170 (residues numbered according to Kabat numbering); or
(c) the protein has reduced glycosylation level compared to a protein comprising the same amino acid sequence except that: the Cα domain comprising serine at position 139, threonine at position 150, and alanine at position 190 (residues numbered according to Kabat numbering); and the Cβ domain comprising glutamic acid at position 134, histidine at position 139, aspartic acid at position 155, and serine at position 170 (residues numbered according to Kabat numbering).

19. The protein of any one of claims 1-6, wherein:
(a) the first polypeptide further comprises the transmembrane and intracellular domains of TCR alpha chain; and
(b) the second polypeptide further comprises the transmembrane and intracellular domains of TCR beta chain.

20. The protein of any one of claims 1-19, wherein:
(a) the protein is linked to a detectable label;
(b) the protein is linked to a therapeutic agent; or
(c) the protein is linked to a therapeutic agent which is a cytotoxic agent, an antiinflammatory agent, or an immunostimulatory agent.

21. A nucleic acid encoding a polypeptide of the protein of any one of claims 1-20.

22. A vector comprising the nucleic acid of claim 21.

23. A cell comprising the nucleic acid of claim 21 or the vector of claim 22.

24. A pharmaceutical composition comprising the protein of any one of claims 1-20, the nucleic acid of claim 21, the vector of claim 22, or the cell of claim 23.

25. The protein of any one of claims 1-20, the nucleic acid of claim 21, the vector of claim 22, the cell of claim 23, or the pharmaceutical composition of claim 24, for use in the treatment of cancer or infection.

## Patentansprüche

1. Protein, umfassend:
ein erstes Polypeptid, umfassend eine T-Zell-Rezeptor(TCR)-Alpha-konstante-Domäne (Cα), umfassend mindestens einen der folgenden Reste: Phenylalanin an Position 139, Isoleucin an Position 150, Threonin an Position 190 (Reste nummeriert gemäß Kabat-Nummerierung); und/oder
ein zweites Polypeptid, umfassend eine TCR-Beta-konstante-Domäne (Cβ), umfassend mindestens einen der folgenden Reste: Arginin an Position 139, Prolin an Position 155, Asparaginsäure oder Glutaminsäure an Position 170 (Reste nummeriert gemäß Kabat-Nummerierung),
wobei das Protein eine höhere Entfaltungstemperatur (Tm) im Vergleich zu einem Protein aufweist, umfassend die gleiche Aminosäuresequenz, außer dass:
die Cα-Domäne Serin an Position 139, Threonin an Position 150 und Alanin an Position 190 (Reste nummeriert gemäß Kabat-Nummerierung) umfasst; und/oder
die Cβ-Domäne Glutaminsäure an Position 134, Histidin an Position 139, Asparaginsäure an Position 155 und Serin an Position 170 (Reste nummeriert gemäß Kabat-Nummerierung) umfasst.

2. Protein nach Anspruch 1, wobei:
(a) das erste Polypeptid eine Cα umfasst, umfassend mindestens einen der folgenden Reste: Phenylalanin an Position 139, Isoleucin an Position 150, Threonin an Position 190 (Reste nummeriert gemäß Kabat-Nummerierung); und das zweite Polypeptid eine Cβ umfasst, umfassend mindestens einen der folgenden Reste: Lysin an Position 134, Arginin an Position 139, Prolin an Position 155, Asparaginsäure oder Glutaminsäure an Position 170 (Reste nummeriert gemäß Kabat-Nummerierung);
(b) das erste Polypeptid eine Cα-Domäne umfasst, umfassend die folgenden Reste: Phenylalanin an Position 139, Isoleucin an Position 150, Threonin an Position 190 (Reste nummeriert gemäß Kabat-Nummerierung); und das zweite Polypeptid eine Cβ-Domäne umfasst, umfassend die folgenden Reste: Lysin an Position 134, Arginin an Position 139, Prolin an Position 155, Asparaginsäure an Position 170 (Reste nummeriert gemäß Kabat-Nummerierung);
oder
(c) das erste Polypeptid eine Cα-Domäne umfasst, umfassend die folgenden Reste: Phenylalanin an Position 139 und Threonin an Position 190 (Reste nummeriert gemäß Kabat-Nummerierung); und das zweite Polypeptid eine Cß-Domäne umfasst, umfassend die folgenden Reste: Lysin an Position 134, Arginin an Position 139, Prolin an Position 155, Asparaginsäure an Position 170 (Reste nummeriert gemäß Kabat-Nummerierung).

3. Protein nach einem der Ansprüche 1 oder 2, wobei:
(a) das erste Polypeptid mit dem zweiten Polypeptid durch eine Disulfidbrücke verknüpft ist; und/oder
(b) die Cα-Domäne ferner einen Cysteinrest an Position 166 (Rest nummeriert gemäß Kabat-Nummerierung) umfasst, und die Cβ-Domäne ferner einen Cysteinrest an Position 173 (Rest nummeriert gemäß Kabat-Nummerierung) umfasst, wobei das erste Polypeptid und das zweite Polypeptid durch eine Disulfidbrücke zwischen dem Cysteinrest an Position 166 von Cα und dem Cysteinrest an Position 173 von Cβ verknüpft sind.

4. Protein nach Anspruch 2 oder 3, wobei:
(a) die Cα-Domäne SEQ ID NO: 5 umfasst und die Cβ-Domäne SEQ ID NO: 7 umfasst;
(b) die Cα-Domäne aus SEQ ID NO: 5 besteht und die Cβ-Domäne aus SEQ ID NO: 7 besteht;
(c) die Cα-Domäne SEQ ID NO: 6 umfasst und die Cβ-Domäne SEQ ID NO: 8 umfasst; oder
(d) die Cα-Domäne aus SEQ ID NO: 6 besteht und die Cβ-Domäne aus SEQ ID NO: 8 besteht.

5. Protein nach einem der Ansprüche 1 bis 4, wobei:
das erste Polypeptid ferner eine TCR-Alpha-variable Domäne (Vα) umfasst; und das zweite Polypeptid ferner eine TCR-Beta-variable Domäne (Vβ) umfasst, wobei die Vα und Vβ eine Antigenbindungsdomäne ausbilden, die ein Antigen bindet.

6. Protein nach Anspruch 5, wobei:
(a) die Vα an den N-Terminus von Cα fusioniert ist und die Vβ an den N-Terminus von Cβ fusioniert ist; und/oder
(b) das Antigen ein Tumorantigen oder ein virales Antigen ist.

7. Protein nach einem der Ansprüche 1 bis 6, wobei das Protein ferner eine zweite Antigenbindungsdomäne umfasst.

8. Protein nach Anspruch 7, wobei:
(a) die zweite Antigenbindungsdomäne an ein Antigen auf der T-ZellOberfläche bindet; oder
(b) die zweite Antigenbindungsdomäne an CD3 bindet.

9. Protein nach Anspruch 7 oder 8, wobei:
(a) die zweite Antigenbindungsdomäne eine scFv, Fab, Fab', (Fab')₂, Einzeldomänenantikörper oder Camelid-VHH-Domäne ist; oder
(b) die zweite Antigenbindungsdomäne ein Fab ist.

10. Protein nach Anspruch 9, wobei die zweite Antigenbindungsdomäne ein Fab ist, die ein Fab einer schweren Kette, umfassend eine variable Domäne (VH) der schweren Kette und eine humane IgG-CH1-Domäne, und ein Fab einer leichten Kette umfasst, umfassend eine variable Domäne der leichten Kette (VL) und eine humane konstante Domäne der leichten Kette (CL), wobei die VH und VL die zweite Antigenbindungsdomäne ausbilden, die CD3 bindet.

11. Protein nach Anspruch 9 oder 10, wobei die zweite Antigenbindungsdomäne ein Fab ist und das Protein drei Polypeptide umfasst:
ein erstes Polypeptid, umfassend Vα - Cα - Linker - VH - CH1;
ein zweites Polypeptid, umfassend Vβ - Cβ; und
ein drittes Polypeptid, umfassend VL - CL;
wobei das zweite und das dritte Polypeptid durch Disulfidbrücken mit dem ersten Polypeptid verknüpft sind.

12. Protein nach einem der Ansprüche 1 bis 11, wobei:
(a) das Protein ferner eine humane IgG-Fc-Region (Fc) umfasst; oder
(b) das Protein ferner eine humane IgG-Fc-Region umfasst, die eine modifizierte humane IgG-Fc-Region mit reduzierter Effektorfunktion im Vergleich zu der entsprechenden humanen IgG-Fc-Region vom Wildtyp ist.

13. Protein nach einem der Ansprüche 1 bis 10 oder 12, wobei das Protein vier Polypeptide umfasst:
ein erstes Polypeptid, umfassend Vα - Cα - Gelenk - erste Fc-Region;
ein zweites Polypeptid, umfassend Vβ - Cβ;
ein drittes Polypeptid, umfassend VL - CL; und
ein viertes Polypeptid, umfassend VH - CH1 - Gelenk - zweite Fc-Region;
wobei das zweite und das vierte Polypeptid durch Disulfidbrücken mit dem ersten Polypeptid verknüpft sind; und das dritte Polypeptid mit dem vierten Polypeptid durch Disulfidbrücken verknüpft ist.

14. Protein nach Anspruch 12, wobei das Protein vier Polypeptide umfasst:
ein erstes Polypeptid, umfassend Vα - Cα - Linker - VH- CH1 - Gelenk - erste Fc-Region;
ein zweites Polypeptid, umfassend Vβ - Cβ;
ein drittes Polypeptid, umfassend VL-CL;
ein viertes Polypeptid, umfassend eine zweite Fc-Region; und
wobei das zweite, das dritte und das vierte Polypeptid durch Disulfidbrücken mit dem ersten Polypeptid verknüpft sind.

15. Protein nach Anspruch 11 oder 14, wobei der Linker eine Aminosäuresequenz umfasst, die aus einer beliebigen von SEQ ID NOs: 41-46 ausgewählt ist.

16. Protein nach Anspruch 13 oder 14, wobei:
(a) die Gelenkregion SEQ ID NO: 47 umfasst und die erste und die zweite Fc-Region SEQ ID NO: 48 umfasst;
(b) die Gelenkregion SEQ ID NO: 49 umfasst und die erste und die zweite Fc-Region SEQ ID NO: 50 umfasst;
(c) die erste und die zweiten Fc-Region einen Satz von CH3-Heterodimerisierungsmutationen umfassen; oder
(d) die erste und die zweite Fc-Region einen Satz von CH3-Heterodimerisierungsmutationen umfassen, wobei eine der ersten oder der zweiten Fc-Region eine CH3-Domäne umfasst, umfassend ein Alanin an Rest 407, ein Methionin an Rest 399 und eine Asparaginsäure an Rest 360; und die andere der ersten oder der zweiten Fc-Region eine CH3-Domäne umfasst, umfassend ein Valin an Rest 366, ein Valin an Rest 409 und ein Arginin an den Resten 345 und 347 (Reste nummeriert gemäß der EU-Index-Nummerierung).

17. Protein nach einem der Ansprüche 1 bis 16, wobei das Protein ein lösliches Protein ist.

18. Protein nach einem der Ansprüche 1 bis 17, wobei:
(a) das Protein eine erhöhte Stabilität im Vergleich zu einem Protein aufweist, umfassend die gleiche Aminosäuresequenz, außer dass: die Cα-Domäne umfassend Serin an Position 139, Threonin an Position 150 und Alanin an Position 190 (Reste nummeriert gemäß Kabat-Nummerierung); und die Cβ-Domäne umfassend Glutaminsäure an Position 134, Histidin an Position 139, Asparaginsäure an Position 155 und Serin an Position 170 (Reste nummeriert gemäß Kabat-Nummerierung);
(b) wenn unter der gleichen Bedingung exprimiert, das Protein eine erhöhte Expressionshöhe im Vergleich zu einem Protein aufweist, umfassend die gleiche Aminosäuresequenz, außer dass: die Cα-Domäne umfassend Serin an Position 139, Threonin an Position 150 und Alanin an Position 190 (Reste nummeriert gemäß Kabat-Nummerierung); und die Cβ-Domäne umfassend Glutaminsäure an Position 134, Histidin an Position 139, Asparaginsäure an Position 155 und Serin an Position 170 (Reste nummeriert gemäß Kabat-Nummerierung); oder
(c) das Protein einen reduzierten Glykosylierungsgrad im Vergleich zu einem Protein aufweist, umfassend die gleiche Aminosäuresequenz, außer dass: die Cα-Domäne umfassend Serin an Position 139, Threonin an Position 150 und Alanin an Position 190 (Reste nummeriert gemäß Kabat-Nummerierung); und die Cβ-Domäne umfassend Glutaminsäure an Position 134, Histidin an Position 139, Asparaginsäure an Position 155 und Serin an Position 170 (Reste nummeriert gemäß Kabat-Nummerierung).

19. Protein nach einem der Ansprüche 1 bis 6, wobei:
(a) das erste Polypeptid ferner die Transmembran- und intrazellulären Domänen der TCR-Alpha-Kette umfasst; und
(b) das zweite Polypeptid ferner die Transmembran- und intrazellulären Domänen der TCR-Beta-Kette umfasst.

20. Protein nach einem der Ansprüche 1 bis 19, wobei:
(a) das Protein mit einer nachweisbare Markierung verknüpft ist;
(b) das Protein mit einem therapeutischen Mittel verknüpft ist; oder
(c) das Protein mit einem therapeutischen Mittel verknüpft ist, das ein zytotoxisches Mittel, ein entzündungshemmendes Mittel oder ein immunstimulierendes Mittel ist.

21. Nukleinsäure, die ein Polypeptid des Proteins nach einem der Ansprüche 1 bis 20 codiert.

22. Vektor, umfassend die Nukleinsäure nach Anspruch 21.

23. Zelle, umfassend die Nukleinsäure nach Anspruch 21 oder den Vektor nach Anspruch 22.

24. Pharmazeutische Zusammensetzung, umfassend das Protein nach einem der Ansprüche 1 bis 20, die Nukleinsäure nach Anspruch 21, den Vektor nach Anspruch 22 oder die Zelle nach Anspruch 23.

25. Protein nach einem der Ansprüche 1 bis 20, die Nukleinsäure nach Anspruch 21, der Vektor nach Anspruch 22, die Zelle nach Anspruch 23 oder die pharmazeutische Zusammensetzung nach Anspruch 24 zur Verwendung bei der Behandlung von Krebs oder Infektion.

## Revendications

1. Protéine comprenant :
un premier polypeptide comprenant un domaine constant alpha (Cα) de récepteur de cellules T (TCR) comprenant au moins l'un des résidus suivants : phénylalanine à la position 139, isoleucine à la position 150, thréonine à la position 190 (résidus numérotés selon la numérotation de Kabat) ; et/ou
un deuxième polypeptide comprenant domaine constant bêta (Cβ) de TCR comprenant au moins l'un des résidus suivants : arginine à la position 139, proline à la position 155, acide aspartique ou acide glutamique à la position 170 (résidus numérotés selon la numérotation de Kabat),
dans laquelle la protéine a une température de dépliage (Tm) plus élevée par comparaison avec une protéine comprenant la même séquence d'acides aminés si ce n'est que :
le domaine Cα comprend sérine à la position 139, thréonine à la position 150 et alanine à la position 190 (résidus numérotés selon la numérotation de Kabat) ; et/ou
le domaine Cβ comprend acide glutamique à la position 134, histidine à la position 139, acide aspartique à la position 155, et sérine à la position 170 (résidus numérotés selon la numérotation de Kabat).

2. Protéine selon la revendication 1, dans laquelle :
(a) le premier polypeptide comprend un Cα comprenant au moins l'un des résidus suivants : phénylalanine à la position 139, isoleucine à la position 150, thréonine à la position 190 (résidus numérotés selon la numérotation de Kabat) ; et le deuxième polypeptide comprend un Cβ comprenant au moins l'un des résidus suivants : lysine à la position 134, arginine à la position 139, proline à la position 155, acide aspartique ou acide glutamique à la position 170 (résidus numérotés selon la numérotation de Kabat) ;
(b) le premier polypeptide comprend un domaine Cα comprenant les résidus suivants : phénylalanine à la position 139, isoleucine à la position 150, thréonine à la position 190 (résidus numérotés selon la numérotation de Kabat) ; et le deuxième polypeptide comprend un domaine Cβ comprenant les résidus suivants : lysine à la position 134, arginine à la position 139, proline à la position 155, acide aspartique à la position 170 (résidus numérotés selon la numérotation de Kabat) ; ou
(c) le premier polypeptide comprend un domaine Cα comprenant les résidus suivants : phénylalanine à la position 139 et thréonine à la position 190 (résidus numérotés selon la numérotation de Kabat) ; et le deuxième polypeptide comprend un domaine Cβ comprenant les résidus suivants : lysine à la position 134, arginine à la position 139, proline à la position 155, acide aspartique à la position 170 (résidus numérotés selon la numérotation de Kabat).

3. Protéine selon l'une quelconque revendication 1 ou 2, dans laquelle :
(a) le premier polypeptide est lié au deuxième polypeptide par une liaison disulfure intercaténaire ; et/ou
(b) le domaine Cα comprend en outre un résidu cystéine à la position 166 (résidu numéroté selon la numérotation de Kabat), et le domaine Cβ comprend en outre un résidu cystéine à la position 173 (résidu numéroté selon la numérotation de Kabat), dans laquelle le premier polypeptide et le deuxième polypeptide sont liés par une liaison disulfure intercaténaire entre le résidu cystéine à la position 166 de Cα et le résidu cystéine à la position 173 de Cβ.

4. Protéine selon la revendication 2 ou 3, dans laquelle :
(a) le domaine Cα comprend SEQ ID NO: 5 et le domaine Cβ comprend SEQ ID NO: 7 ;
(b) le domaine Cα est constitué de SEQ ID NO: 5 et le domaine Cβ est constitué de SEQ ID NO: 7 ;
(c) le domaine Cα comprend SEQ ID NO: 6 et le domaine Cβ comprend SEQ ID NO: 8 ; ou
(d) le domaine Cα est constitué de SEQ ID NO: 6 et le domaine Cβ est constitué de SEQ ID NO: 8.

5. Protéine selon l'une quelconque des revendications 1 à 4, dans laquelle :
le premier polypeptide comprend en outre un domaine variable alpha (Vα) de TCR ; et
le deuxième polypeptide comprend en outre un domaine variable bêta (Vβ) de TCR,
dans laquelle les Vα et Vβ forment un domaine de liaison d'antigène qui lie un antigène.

6. Protéine selon la revendication 5, dans laquelle :
(a) le Vα est fusionné à la terminaison N de Cα et le Vβ est fusionné à la terminaison N de Cβ ; et/ou
(b) l'antigène est un antigène tumoral ou un antigène viral.

7. Protéine selon l'une quelconque des revendications 1 à 6, dans laquelle la protéine comprend en outre un second domaine de liaison d'antigène.

8. Protéine selon la revendication 7, dans laquelle :
(a) le second domaine de liaison d'antigène lie un antigène sur une surface de cellule T ; ou
(b) le second domaine de liaison d'antigène lie CD3.

9. Protéine selon la revendication 7 ou 8, dans laquelle :
(a) le second domaine de liaison d'antigène est un scFv, un Fab, un Fab', un (Fab')₂, un anticorps à domaine unique, ou un domaine VHH de camélidé ; ou
(b) le second domaine de liaison d'antigène est un Fab.

10. Protéine selon la revendication 9, dans laquelle le second domaine de liaison d'antigène est un Fab qui comprend une chaîne lourde de Fab comprenant un domaine variable de chaîne lourde (VH) et un domaine CH1 d'IgG humaine, et une chaîne légère de Fab comprenant un domaine variable de chaîne légère (VL) et un domaine constant de chaîne légère humaine (CL), dans laquelle les VH et VL forment le second domaine de liaison d'antigène qui lie CD3.

11. Protéine selon la revendication 9 ou 10, dans laquelle le second domaine de liaison d'antigène est un Fab et la protéine comprend trois polypeptides :
un premier polypeptide comprenant Vα - Cα - séquence de liaison - VH - CH1 ;
un deuxième polypeptide comprenant Vβ - Cβ ; et
un troisième polypeptide comprenant VL - CL ;
dans laquelle les deuxième et troisième polypeptides sont liés au premier polypeptide par une liaison disulfure intercaténaire.

12. Protéine selon l'une quelconque des revendications 1 à 11, dans laquelle :
(a) la protéine comprend en outre une région Fc d'IgG humaine (Fc) ; ou
(b) la protéine comprend en outre une région Fc d'IgG humaine qui est une région Fc d'IgG humaine modifiée avec une fonction effectrice réduite par comparaison avec la région Fc d'IgG humaine de type sauvage correspondante.

13. Protéine selon l'une quelconque des revendications 1 à 10, ou 12, dans laquelle la protéine comprend quatre polypeptides :
un premier polypeptide comprenant Vα - Cα - charnière - première région Fc ;
un deuxième polypeptide comprenant Vβ - Cβ ;
un troisième polypeptide comprenant VL - CL ; et
un quatrième polypeptide comprenant VH - CH1 - charnière - seconde région Fc ;
dans laquelle les deuxième et quatrième polypeptides sont liés au premier polypeptide par une liaison disulfure intercaténaire ; et le troisième polypeptide est lié au quatrième polypeptide par des liaisons disulfure intercaténaires.

14. Protéine selon la revendication 12, dans laquelle la protéine comprend quatre polypeptides :
un premier polypeptide comprenant Vα - Cα - séquence de liaison - VH-CH1 - charnière - première région Fc ;
un deuxième polypeptide comprenant Vβ - Cβ ;
un troisième polypeptide comprenant VL - CL ;
un quatrième polypeptide comprenant une seconde région Fc ; et
dans laquelle les deuxième, troisième et quatrième polypeptides sont liés au premier polypeptide par une liaison disulfure intercaténaire.

15. Protéine selon la revendication 11 ou 14, dans laquelle la séquence de liaison comprend une séquence d'acides aminés choisie parmi l'une quelconque parmi les SEQ ID NO: 41 à 46.

16. Protéine selon la revendication 13 ou 14, dans laquelle :
(a) la région charnière comprend SEQ ID NO: 47 et les première et seconde régions Fc comprennent SEQ ID NO: 48 ;
(b) la région charnière comprend SEQ ID NO: 49 et les première et seconde régions Fc comprennent SEQ ID NO: 50 ;
(c) les première et seconde régions Fc comprennent un ensemble de mutations d'hétérodimérisation de CH3 ; ou
(d) les première et seconde régions Fc comprennent un ensemble de mutations d'hétérodimérisation de CH3, dans laquelle l'une de la première ou de la seconde région Fc comprend un domaine CH3 comprenant une alanine au niveau du résidu 407, une méthionine au niveau du résidu 399 et un acide aspartique au niveau du résidu 360 ; et l'autre de ladite première ou seconde région Fc comprend un domaine CH3 comprenant une valine au niveau du résidu 366, une valine au niveau du résidu 409 et une arginine au niveau des résidus 345 et 347 (résidus numérotés selon la numérotation d'index EU).

17. Protéine selon l'une quelconque des revendications 1 à 16, dans laquelle la protéine est une protéine soluble.

18. Protéine selon l'une quelconque des revendications 1 à 17, dans laquelle :
(a) la protéine a une stabilité accrue par comparaison avec une protéine comprenant la même séquence d'acides aminés si ce n'est que : le domaine Cα comprend sérine à la position 139, thréonine à la position 150 et alanine à la position 190 (résidus numérotés selon la numérotation de Kabat) ; et le domaine Cβ comprend acide glutamique à la position 134, histidine à la position 139, acide aspartique à la position 155 et sérine à la position 170 (résidus numérotés selon la numérotation de Kabat) ;
(b) lorsqu'elle est exprimée dans la même condition, la protéine a un niveau d'expression accru par comparaison avec une protéine comprenant la même séquence d'acides aminés si ce n'est que : le domaine Cα comprend sérine à la position 139, thréonine à la position 150 et alanine à la position 190 (résidus numérotés selon la numérotation de Kabat) ; et le domaine Cβ comprend acide glutamique à la position 134, histidine à la position 139, acide aspartique à la position 155 et sérine à la position 170 (résidus numérotés selon la numérotation de Kabat) ; ou
(c) la protéine a un niveau de glycosylation réduit par comparaison avec une protéine comprenant la même séquence d'acides aminés si ce n'est que : le domaine Cα comprend sérine à la position 139, thréonine à la position 150 et alanine à la position 190 (résidus numérotés selon la numérotation de Kabat) ; et le domaine Cβ comprend acide glutamique à la position 134, histidine à la position 139, acide aspartique à la position 155 et sérine à la position 170 (résidus numérotés selon la numérotation de Kabat).

19. Protéine selon l'une quelconque des revendications 1 à 6, dans laquelle :
(a) le premier polypeptide comprend en outre les domaines transmembranaires et intracellulaires de chaîne alpha de TCR ; et
(b) le deuxième polypeptide comprend en outre les domaines transmembranaires et intracellulaires de chaîne bêta de TCR.

20. Protéine selon l'une quelconque des revendications 1 à 19, dans laquelle :
(a) la protéine est liée à un marqueur détectable ;
(b) la protéine est liée à un agent thérapeutique ; ou
(c) la protéine est liée à un agent thérapeutique qui est un agent cytotoxique, un agent anti-inflammatoire, ou un agent immunostimulateur.

21. Acide nucléique codant pour un polypeptide de la protéine selon l'une quelconque des revendications 1 à 20.

22. Vecteur comprenant l'acide nucléique selon la revendication 21.

23. Cellule comprenant l'acide nucléique selon la revendication 21 ou le vecteur selon la revendication 22.

24. Composition pharmaceutique comprenant la protéine selon l'une quelconque des revendications 1 à 20, l'acide nucléique selon la revendication 21, le vecteur selon la revendication 22, ou la cellule selon la revendication 23.

25. Protéine selon l'une quelconque des revendications 1 à 20, acide nucléique selon la revendication 21, vecteur selon la revendication 22, cellule selon la revendication 23, ou composition pharmaceutique selon la revendication 24, pour utilisation dans le traitement d'un cancer ou d'une infection.
